# EUROPEAN PATENT SPECIFICATION

(11) **EP 3 348 650 B1**
(45) Date of publication and mention of the grant of the patent: **23.12.2020**
(21) Application number: 16911701.7
(22) Date of filing: 05.08.2016
(51) Int. Cl.: C12Q 1/68

(54) **KIT AND METHOD FOR DETECTING SINGLE NUCLEOTIDE POLYMORPHISM**
KIT UND VERFAHREN ZUM NACHWEIS VON EINZELNUKLEOTID-POLYMORPHISMUS
KIT ET PROCÉDÉ DE DÉTECTION DE POLYMORPHISME NUCLÉOTIDIQUE SIMPLE

(43) Date of publication of application: 18.07.2018
(73) Proprietor: Heimbiotek Inc., Hwaseong-si, Gyeonggi-do 18384 (KR)
(72) Inventor: LEE, Jae Hoon, Guri-si Gyeonggi-do 11935 (KR)
(74) Representative: Isarpatent
(86) International application number: PCT/KR2016/008669
(87) International publication number: WO 2018/026039

(56) References cited:
- WO-A1-03/020983
- JP-A- 2010 517 545
- JP-A- 2013 070 639
- KR-A- 20040 088 538
- KR-A- 20120 046 018
- KR-A- 20120 047 767
- KR-A- 20160 098 620
- MURIEL GAUDET ET AL: "Allele specific pcr in SNP genotyping", 1 January 2009 (2009-01-01), SINGLE NUCLEOTIDE POLYMORPHISMS : METHODS AND PROTO, HUMANA PR, USA, PAGE(S) 415 - 423, XP009177497, ISBN: 978-1-60327-410-4 * Fig. 26.1 and 26.2 *
- SOBRINO B ET AL: "SNPs in forensic genetics: a review on SNP typing methodologies", FORENSIC SCIENCE INTERNATIONAL, ELSEVIER B.V, AMSTERDAM, NL, vol. 154, no. 2-3, 25 November 2005 (2005-11-25), pages 181-194, XP027757784, ISSN: 0379-0738 [retrieved on 2005-11-25]
- LIU QIANG ET AL: "Overlapping PCR for bidirectional PCR amplification of specific alleles: A rapid one-tube method for simultaneously differentiating homozygotes and heterozygotes", PCR METHODS & APPLICATIONS, COLD SPRING HARBOR LABORATORY PRESS, US, vol. 7, no. 4, 1 January 1997 (1997-01-01) , pages 389-398, XP002259043, ISSN: 1054-9803

## Description

### TECHNICAL FIELD

The present invention relates to a kit and a method for detecting DNA, and more particularly to a kit and a method for detecting single nucleotide polymorphism.

### BACKGROUND ART

Since the human genome map was completed, it has been suggested that the prediction of diseases, the diagnosis and prognosis of diseases and the prediction of response to drugs can be achieved more widely. In recent years, the functions of various genes have been revealed. As the functions of various genes have been identified, the degree of risk of developing a disease can be predicted, for example, by analyzing a mutation of a certain gene in a person, whereby the disease can be prevented in advance. Furthermore, through the genetic mutation analysis of infectious agents (e.g., virus) that cause infections in living organisms, it is possible to analyze the virus has resistance to a certain therapeutic drug. Through the aforesaid genotype analysis, the response of an infectious agent to a specific therapeutic drug, for example, a resistance response, can be predicted, whereby personalized treatment methods for individual patients can be developed. When a new PCR technology is applied to single nucleotide polymorphism (SNP) analysis which is the core technology of such personalized medicine and companion diagnostics in order to analyze SNP, it is possible to solve problems such as the limitations of existing PCR and NGS (Next Generation Sequencing), the necessity of expensive analysis systems, and the consumption of much time until analysis, in personalized medicine and companion diagnostics for determining drug efficacy according to the patient's genotype. Specifically, existing PCR (e.g., AS-PCR based) or the like has limitations such as difficulty in analysis of heterozygous point mutations, and real-time PCR employing TaqMan probes or the like has disadvantages in that analysis channels are limited due to the limitation of light sources and fluorescent substances and in that the development of point-of-care-testing (POCT) devices is difficult. Next-generation sequencing (NGS) requires high analysis costs, expensive systems, and complicated processes, is time-consuming, and cannot also be applied to POCT.

International application WO 03/020983 A1 discloses a methodology for a two-level allele-specific extension reaction for use in ultra-high throughput genotyping. A primary PCR reaction is carried out with primers containing both sequence-specific and artificial, universal domains. The primers are designed to render the PCR products allele-specific and amenable to amplification with secondary primers which also contain universal domains. The secondary primers are designed to maintain allelespecificity, and to provide a detectable label.

The article "Allele-Specific PCR in SNP Genotyping" by Muriel Gaudet et al., in "Single Nucleotide Polymorphisms - Methods and Protocols", Anton A. Komar (Ed.), Humana Press 2009, discloses allele-specific PCR in a single reaction with standard PCR conditions. A common reverse primer and two forward allele-specific primers with different tails amplify two allele-specific PCR products of different lengths, which are further separated by agarose gel electrophoresis. PCR specificity is improved by the introduction of a destabilizing mismatch within the 3' end of the allele-specific primers.

### SUMMARY OF INVENTION

The present invention has been made in order to solve various problems, including the above-described problems, and it is an object of the present invention to provide a novel kit and method for detecting single nucleotide polymorphism, which can be easily developed into a point-of-care-testing (POCT) device.

In accordance with one aspect of the present invention, there is provided a kit for detecting single nucleotide polymorphism, comprising:
(a1) a single nucleotide polymorphic first allele-specific forward primer comprising:
   a 5'-end first tag oligonucleotide which does not hybridize to a target nucleic acid under PCR conditions; and a single nucleotide polymorphic first allele-specific oligonucleotide which is capable of hybridizing specifically to the target nucleic acid and has at its 3'-end, a nucleotide specific for the first allele of the single nucleotide polymorphism, to generate a first PCR product;
(a2') a single nucleotide polymorphic second allele-specific forward primer comprising:
   a 5'-end second tag oligonucleotide which does not hybridize to the target nucleic acid and the 5'-end first tag oligonucleotide under PCR conditions; and a single nucleotide polymorphic second allele-specific oligonucleotide which is capable of hybridizing specifically to the target nucleic acid and has at its 3'-end, a nucleotide specific for the second allele of the single nucleotide polymorphism, to generate a PCR second product, or optionally
(a2") a single nucleotide polymorphic second allele-specific forward primer comprising a single nucleotide polymorphic second allele-specific oligonucleotide which is capable of hybridizing specifically to the target nucleic acid and has at its 3'-end, a nucleotide specific for the second allele of the single nucleotide polymorphism, to generate a PCR second product; and
(b) a universal reverse primer for amplifying the target nucleic acid; and
(c1) a first extension primer hybridizing to the complementary of the 5'-end first tag oligonucleotide comprising:
   c11) a 5'-end first extension polynucleotide which does not hybridize to any of the target nucleic acid, the single nucleotide polymorphic first allele-specific forward primer and the reverse primer under PCR conditions, wherein the first extension polynucleotide is 20 to 200 nt and provided for extending the length of the first PCR product; and
   c12) a 3'-end first tag oligonucleotide which hybridizes to the complementary of the 5'-end first tag oligonucleotide of the first PCR product.

Preferably, the kit of the present invention, further comprises:
(c2) a second extension primer hybridizing to the complementary of the 5'-end second tag oligonucleotide comprising:
c21) a 5'-end second extension polynucleotide which does not hybridize to any of the target nucleic acid, the single nucleotide polymorphic first allele-specific forward primer, the single nucleotide polymorphic second allele-specific forward primer and the reverse primer under PCR conditions, the second extension polynucleotide having a length which is distinguishable from that of the first extension polynucleotide in gel electrophoresis, and wherein the second extension polynucleotide is provided for extending the length of the second PCR product; and
c22) a 3'-end second tag oligonucleotide which hybridizes to the complementary of the 5'- end second tag oligonucleotide of the second PCR product.

Preferably, the kit for detecting single nucleotide polymorphism in the present invention comprises:
a1) a wild-type allele-specific forward primer comprising: a 5'-end first tag oligonucleotide which does not hybridize to a target nucleic acid under PCR conditions; and a wild-type allele-specific oligonucleotide which is capable of hybridizing specifically to the target nucleic acid and has at its 3'-end, a nucleotide specific for a wild-type allele of the single nucleotide polymorphism, to generate a first PCR product;
a2') a mutant allele-specific forward primer comprising: a 5'-end second tag oligonucleotide which does not hybridize to the target nucleic acid and the 5'-end first tag oligonucleotide under PCR conditions; and a mutant allele-specific oligonucleotide which is capable of hybridizing specifically to the target nucleic acid and has at its 3'-end, a nucleotide specific for a mutant allele of the single nucleotide polymorphism, to generate a second PCR product, or optionally
a2") a mutant allele-specific forward primer comprising a mutant allele-specific oligonucleotide which is capable of hybridizing specifically to the target nucleic acid and has at its 3'-end, a nucleotide specific for the mutant allele of the single nucleotide polymorphism, to generate a second PCR product;
b) a universal reverse primer for amplifying the target nucleic acid;
c1) a first extension primer hybridizing to the complementary of the 5'-end first tag oligonucleotide comprising:
   c11) a 5'-end first extension polynucleotide which does not hybridize to any of the target nucleic acid, the wild-type allele-specific forward primer, the mutant allele-specific forward primer and the reverse primer under PCR conditions, wherein the first extension primer is 20 to 200 nt and provided for extending the length of the first PCR product, and
   c12) a 3'-end first tag oligonucleotide which hybridizes to the complementary of the 5'-end first tag oligonucleotide of the first PCR product;
   c2) a second extension primer hybridizing to the complementary of the 5'-end second tag oligonucleotide, comprising:
      c21) a 5'-end second extension polynucleotide which does not hybridize to any of the target nucleic acid, the wild-type allele-specific forward primer, the mutant allele-specific forward primer and the reverse primer under PCR conditions, the second extension polynucleotide having a length which is distinguishable from that of the first extension polynucleotide in gel electrophoresis, and wherein the second extension polynucleotide is provided for extending the length of the second PCR product; and
      c22) a 3'-end second tag oligonucleotide which hybridizes to the complementary of the 5'-end second tag oligonucleotide of the second PCR product.

Preferably, the kit of the present invention further comprises:
a1) a wild-type allele-specific forward primer comprising: a 5'-end first tag oligonucleotide which does not hybridize to a target nucleic acid under PCR conditions; and a wild-type allele-specific oligonucleotide which is capable of hybridizing specifically to the target nucleic acid and has at its 3'-end, a nucleotide specific for a wild-type allele of the single nucleotide polymorphism, to generate a first PCR product;
a2') a mutant allele-specific forward primer comprising: a 5'-end second tag oligonucleotide which does not hybridize to the target nucleic acid and the 5'-end first tag oligonucleotide under PCR conditions; and a mutant allele-specific oligonucleotide which is capable of hybridizing specifically to the target nucleic acid and has at its 3'-end, a nucleotide specific for a mutant allele of the single nucleotide polymorphism, to generate a second PCR product, or optionally
a2") a mutant allele-specific forward primer comprising a mutant allele-specific oligonucleotide which is capable of hybridizing specifically to the target nucleic acid and has at its 3'-end, a nucleotide specific for the mutant allele of the single nucleotide polymorphism, to generate a second PCR product;
b) a universal reverse primer for amplifying the target nucleic acid;
c1) a first extension primer hybridizing to the complementary of the 5'-end first tag oligonucleotide comprising:
   c11) a 5'-end first extension polynucleotide which does not hybridize to any of the target nucleic acid, the wild-type allele-specific forward primer, the mutant allele-specific forward primer and the reverse primer under PCR conditions, wherein the first extension primer is 20 to 200 nt and provided for extending the length of the first PCR product, and
   c12) a 3'-end first tag oligonucleotide which hybridizes to the complementary of the 5'-end first tag oligonucleotide of the first PCR product;
c2) a second extension primer hybridizing to the complementary of the 5'-end second tag oligonucleotide, comprising:
   c21) a 5'-end second extension polynucleotide which does not hybridize to any of the target nucleic acid, the wild-type allele-specific forward primer, the mutant allele-specific forward primer and the reverse primer under PCR conditions, the second extension polynucleotide having a length which is distinguishable from that of the first extension polynucleotide in gel electrophoresis, and wherein the second extension polynucleotide is provided for extending the length of the second PCR product; and
   c22) a 3'-end second tag oligonucleotide which hybridizes to the complementary of the 5'-end second tag oligonucleotide of the second PCR product; and
d) an extension product-amplification forward primer comprising a consensus sequence from the first extension polynucleotide and the second extension polynucleotide.

In accordance with still another aspect of the present invention, there is provided a first method for detecting single nucleotide polymorphism, comprising:
(i) adding, to a reaction solution comprising a target nucleic acid sample to be amplified, a dNTP mixture, a thermally stable DNA polymerase and a reaction buffer,
(a1) a single nucleotide polymorphic first allele-specific forward primer comprising:
   a 5'-end first tag oligonucleotide which does not hybridize to a target nucleic acid under PCR conditions; and
   a single nucleotide polymorphic first allele-specific oligonucleotide which is capable of hybridizing specifically to the target nucleic acid and has at its 3'-end, a nucleotide specific for the first allele of the single nucleotide polymorphism, to generate a first PCR product;
(a2') a single nucleotide polymorphic second allele-specific forward primer comprising: a 5'-end second tag oligonucleotide which does not hybridize to the target nucleic acid and the 5'-end first tag oligonucleotide under PCR conditions; and a single nucleotide polymorphic second allele-specific oligonucleotide which is capable of hybridizing specifically to the target nucleic acid and has at its 3'-end, a nucleotide specific for the second allele of the single nucleotide polymorphism, to generate a second PCR product, or optionally
(a2") a single nucleotide polymorphic second allele-specific forward primer comprising a single nucleotide polymorphic second allele-specific oligonucleotide which is capable of hybridizing specifically to the target nucleic acid and has at its 3'-end, a nucleotide specific for the second allele of the single nucleotide polymorphism, to generate a second PCR product; and
(b) a universal reverse primer for amplifying the target nucleic acid;
(c1) a first extension primer hybridizing to the complementary of the 5'-end first tag oligonucleotide comprising:
   c11) a 5'-end first extension polynucleotide which does not hybridize to any of the target nucleic acid, the single nucleotide polymorphic first allele-specific forward primer and the reverse primer under PCR conditions, wherein the first extension polynucleotide is 20 to 200 nt and provided for extending the length of the first PCR product; and
   c12) a 3'-end first tag oligonucleotide which hybridizes to the complementary of the 5'-end first tag oligonucleotide of the first PCR product; and
(d1) a first extension product-amplification forward primer comprising a nucleic acid sequence selected from the first extension polynucleotide; and
   (ii) performing PCR of the reaction solution.

Preferably, the above first method for detecting single nucleotide polymorphism in the present invetnion further comprises:
(c2) a second extension primer hybridizing to the complementary of the 5'-end second tag oligonucleotide comprising:
   c21) a 5'-end second extension polynucleotide which does not hybridize to any of the target nucleic acid, the single nucleotide polymorphic first allele-specific forward primer, the single nucleotide polymorphic second allele-specific forward primer and the reverse primer under PCR conditions, the second extension polynucleotide having a length which is distinguishable from that of the first extension polynucleotide in gel electrophoresis, and wherein the second extension polynucleotide is provided for extending the length of the second PCR product; and
   c22) a 3'-end second tag oligonucleotide which hybridizes to the complementary of the 5'-end second tag oligonucleotide of the second PCR product; and
(d2) a second extension product-amplification forward primer comprising a nucleic acid sequence selected from the second extension polynucleotide.In accordance with still another aspect of the present invention, there is provided a second method for detecting single nucleotide polymorphism, comprising:
   (i) performing allele-specific PCR of a target nucleic acid using:
      (a1) a single nucleotide polymorphic first allele-specific forward primer comprising:
         a 5'-end first tag oligonucleotide which does not hybridize to a target nucleic acid under PCR conditions; and
         a single nucleotide polymorphic first allele-specific oligonucleotide which is capable of hybridizing specifically to the target nucleic acid and has at its 3'-end, a nucleotide specific for the first allele of the single nucleotide polymorphism, to generate a first PCR product;
      (a2') a single nucleotide polymorphic second allele-specific forward primer comprising:
         a 5'-end second tag oligonucleotide which does not hybridize to the target nucleic acid and the 5'-end first tag oligonucleotide under PCR conditions; and
         a single nucleotide polymorphic second allele-specific oligonucleotide which is capable of hybridizing specifically to the target nucleic acid and has at its 3'-end, a nucleotide specific for the second allele of the single nucleotide polymorphism, to generate a second PCR product, or optionally
      (a2") a single nucleotide polymorphic second allele-specific forward primer comprising a single nucleotide polymorphic second allele-specific oligonucleotide which is capable of hybridizing specifically to the target nucleic acid and has at its 3'-end, a nucleotide specific for the second allele of the single nucleotide polymorphism, to generate a second PCR product; and
(b) a universal reverse primer for amplifying the target nucleic acid; and
   (ii) performing primer extension of a product of the allele-specific PCR by use of
   (c1) a first extension primer hybridizing to the complementary of the 5'-end first tag oligonucleotide comprising:
      c11) a 5'-end first extension polynucleotide which does not hybridize to any of the target nucleic acid, the single nucleotide polymorphic first allele-specific forward primer and the reverse primer under PCR conditions, wherein the first extension polynucleotide is 20 to 200 nt and provided for extending the length of the first PCR product; and
      c12) a 3'-end first tag oligonucleotide which hybridizes to the complementary of the 5'-end first tag oligonucleotide of the first PCR product.

Preferably, the above secondmethod for detecting single nucleotide polymorphism in the present invention further addes the following to the solution in step (ii):
(c2) a second extension primer hybridizing to the complementary of the 5'-end second tag oligonucleotide comprising:
c21) a 5'-end second extension polynucleotide which does not hybridize to any of the target nucleic acid, the single nucleotide polymorphic first allele-specific forward primer, the single nucleotide polymorphic second allele-specific forward primer and the reverse primer under PCR conditions, the second extension polynucleotide having a length which is distinguishable from that of the first extension polynucleotide in gel electrophoresis, and wherein the second extension polynucleotide is provided for extending the length of the second PCR product; and
c22) a 3'-end second tag oligonucleotide which hybridizes to the complementary of the 5'-end second tag oligonucleotide of the second PCR product.

Preferably, the above second method for detecting single nucleotide polymorphism in the present invention comprises:
i) performing single nucleotide polymorphism-specific PCR of a target nucleic acid using:
   a1) a wild-type allele-specific forward primer comprising: a 5'-end first tag oligonucleotide which does not hybridize to the target nucleic acid under PCR conditions; and a wild-type allele-specific oligonucleotide which is capable of hybridizing specifically to the target nucleic acid and has at its 3'-end, a nucleotide specific for a wild-type allele of the single nucleotide polymorphism, to generate a first PCR product;
   a2') a mutant allele-specific forward primer comprising: a 5'-end second tag oligonucleotide which does not hybridize to the target nucleic acid and the first tag oligonucleotide under PCR conditions; and a mutant allele-specific oligonucleotide which is capable of hybridizing specifically to the target nucleic acid and has at its 3'-end, a nucleotide specific for a mutant allele of the single nucleotide polymorphism, to genearte a second PCR prodcut, or optionally
   a2") a mutant allele-specific forward primer comprising a mutant allele-specific oligonucleotide which is capable of hybridizing specifically to the target nucleic acid and has at its 3'-end, a nucleotide specific for the mutant allele of the single nucleotide polymorphism, to generate a second PCR product; and
   b) a universal reverse primer for amplifying the target nucleic acid; and
ii) performing primer extension of a product of the single nucleotide polymorphism-specific PCR by use of:
   c1) a first extension primer hybridizing to the complementary of the 5'-end first tag oligonucleotide, comprising:
   c11) a 5'-end first extension polynucleotide which does not hybridize to any of the target nucleic acid, the wild-type allele-specific forward primer, the mutant allele-specific forward primer and the reverse primer under PCR conditions, wherein the first extension polynucleotide is 20 to 200 nt and provided for extending the length of the first PCR product; and
   c12) a 3'-end first tag oligonucleotide which hybridizes to the complementary of the 5'-end first tag oligonucleotide of the first PCR product; and
   c2) a second extension primer hybridizing to the complementary of the 5'-end second tag oligonucleotide comprising:
      c21) a 5'-end second extension polynucleotide which does not hybridize to any of the target nucleic acid, the wild-type allele-specific forward primer, the mutant allele-specific forward primer and the reverse primer under PCR conditions, the second extension polynucleotide having a length which is distinguishable from that of the first extension polynucleotide in gel electrophoresis, and wherein the second extension polynucleotide is provided for extending the length of the second PCR product; and
      c22) a 3'-end second tag oligonucleotide which hybridizes to the complementary of the 5'-end second tag oligonucleotide of the second PCR product.

### DETAILED DESCRIPTION OF INVENTION

### Definition of Terms

Hereinafter, the terms used herein will be defined.

As used herein, the term "nucleotide" refers to the monomeric unit of nucleic acid, which is composed of bases, pentose and phosphate. For DNA, the bases are adenine, guanine, cytosine, and thymine, and for RNA, uracil is substituted for thymine. For DNA, the pentose is 2'-deoxyribose, and for RNA, the pentose is ribose.

As used herein, the term "oligonucleotide" generally refers to a polymer of nucleotide monomer units, which is a short single-stranded nucleic acid molecule composed of 13 to 25 nucleotides. In some cases, the term may refer to a nucleic acid molecule composed of less than 13 nucleotides, including 6-mer, 7-mer, 8-mer, 9-mer, 10-mer, 11-mer and 12-mer, or more than 25 nucleotides.

As used herein, the term "polynucleotide" generally refers to a polymer of nucleotide units, which is longer than the oligonucleotide as defined above, but may also be used interchangeably with the term "oligonucleotide". Polynucleotides include both single-stranded and double-stranded nucleic acid molecules.

As used herein, the term "sense strand" refers to one single strand of a double-stranded DNA molecule, which is oriented in the same direction as the direction in which the gene of interest is read, and the term "antisense strand" refers to the other single strand complementary to the sense strand. However, regardless of the direction in which the gene is read, a strand whose nucleic acid sequence is first known may also be defined as "sense strand", and a strand complementary thereto may be defined as "antisense strand".

As used herein, the term "PCR (polymerase chain reaction)" or "nucleic acid amplification reaction" refers to a reaction that amplifies a certain target nucleic acid molecule using a heat-stable DNA polymerase. PCR is performed using a reaction buffer containing, in addition to the DNA polymerase, oligonucleotide primers (forward and reverse primers) capable of hybridizing specifically to a target nucleic acid, a deoxynucleotide (dNTP) mixture, divalent ions such as Mg²⁺, and the like. A DNA molecule produced by the PCR reaction is herein referred to as "amplification product".

As used herein, the term "primer extension" refers to a non-chain reaction in which a template nucleic acid having a limited length is extended using a DNA polymerase and primers complementary thereto and which is terminated at the 5'-end of the template nucleic acid. A DNA molecule produced by the primer extension is herein referred to as "extension product".

As used herein, the term "primer" refers to an oligonucleotide or polynucleotide that hybridizes complementarily to template DNA and that is used to initiate PCR reaction or primer extension. PCR reaction is performed using a forward primer (or a sense primer) selected from a sense strand which is oriented in the same direction as the direction in which a nucleic acid molecule to be amplified is read, and a reverse primer (or an antisense primer) selected from an antisense strand complementary to the sense strand. Primer extension is generally performed using a single extension primer.

As used herein, the term "tag" refers to an oligonucleotide which is attached to the 5'- end of an allele-specific primer in order to specifically distinguish a certain single nucleotide polymorphism. The tag has a specific nucleic acid sequence according to a corresponding single-nucleotide polymorphism nucleotide. The nucleic acid may be designed to have no homology to a target template nucleic acid to be amplified and other primers, except for a primer for extension, and thus may be designed so as to minimize nonspecific amplification.

As used herein, the term "single-nucleotide polymorphism (SNP)" refers to a genetic change or variation reflecting a single difference at a single nucleotide (A, T, G or C) in a DNA nucleotide sequence. Generally, SNP is defined as a genomic locus where two or more alleles present at a frequency of at least 1% in the population occur. A polymorphism with an allele frequency of at least 5% is classified as common polymorphism, and a polymorphism with an allele frequency of 1 to 5% is classified as rare polymorphism.

As used herein, the term "allele" refers to any one of a series of two or more different forms of a gene sequence that can exist at the same genetic locus on a chromosome. Sometimes, other alleles may cause other observable phenotypic traits. However, most genetic mutations hardly cause observable mutations. As used herein, "first allele of single-nucleotide polymorphism" and "second allele of single-nucleotide polymorphism" refer to a plurality of alleles, which occur by base variation(s) at an SNP site and are discriminated in an arbitrary order. When any one of the first allele and the second allele is a major allele (high-frequency allele or wild-type allele), the other may be a minor allele (low-frequency allele or mutant allele).

As used herein, the term "allele-specific bidirectional primer extension (ASBPE)" means an allele-specific bidirectional primer extension reaction capable of detecting the presence or absence and kind of mutation of SNP. It is possible to detect the presence or absence and kind of mutation of SNP by amplifying a nucleic acid molecule extended by the extension reaction, with a pair of primers comprising a nucleic acid sequence selected from an extension primer used in the extension reaction.

### Disclosure of the Invention

In accordance with one aspect of the present invention, there is provided a kit for detecting single nucleotide polymorphism, comprising:
(a1) a single nucleotide polymorphic first allele-specific forward primer comprising:
   a 5'-end first tag oligonucleotide which does not hybridize to a target nucleic acid under PCR conditions; and a single nucleotide polymorphic first allele-specific oligonucleotide which is capable of hybridizing specifically to the target nucleic acid and has at its 3'-end, a nucleotide specific for the first allele of the single nucleotide polymorphism, to generate a first PCR product;
(a2') a single nucleotide polymorphic second allele-specific forward primer comprising:
   a 5'-end second tag oligonucleotide which does not hybridize to the target nucleic acid and the 5'-end first tag oligonucleotide under PCR conditions; and a single nucleotide polymorphic second allele-specific oligonucleotide which is capable of hybridizing specifically to the target nucleic acid and has at its 3'-end, a nucleotide specific for the second allele of the single nucleotide polymorphism, to generate a PCR second product, or optionally
(a2") a single nucleotide polymorphic second allele-specific forward primer comprising a single nucleotide polymorphic second allele-specific oligonucleotide which is capable of hybridizing specifically to the target nucleic acid and has at its 3'-end, a nucleotide specific for the second allele of the single nucleotide polymorphism, to generate a PCR second product; and
(b) a universal reverse primer for amplifying the target nucleic acid; and
(c1) a first extension primer hybridizing to the complementary of the 5'-end first tag oligonucleotide comprising:
   c11) a 5'-end first extension polynucleotide which does not hybridize to any of the target nucleic acid, the single nucleotide polymorphic first allele-specific forward primer and the reverse primer under PCR conditions, wherein the first extension polynucleotide is 20 to 200 nt and provided for extending the length of the first PCR product; and
   c12) a 3'-end first tag oligonucleotide which hybridizes to the complementary of the 5'-end first tag oligonucleotide of the first PCR product.

Preferably, the kit of the present invention, further comprises:
(c2) a second extension primer hybridizing to the complementary of the 5'-end second tag oligonucleotide comprising:
c21) a 5'-end second extension polynucleotide which does not hybridize to any of the target nucleic acid, the single nucleotide polymorphic first allele-specific forward primer, the single nucleotide polymorphic second allele-specific forward primer and the reverse primer under PCR conditions, the second extension polynucleotide having a length which is distinguishable from that of the first extension polynucleotide in gel electrophoresis, and wherein the second extension polynucleotide is provided for extending the length of the second PCR product; and
c22) a 3'-end second tag oligonucleotide which hybridizes to the complementary of the 5'- end second tag oligonucleotide of the second PCR product.

The kit for detecting single nucleotide polymorphism may further comprise iv) an extension product-amplification forward primer comprising a nucleic acid sequence selected from the extension polynucleotide. The kit for detecting single nucleotide polymorphism may further comprise (d1) a first extension product-amplification forward primer comprising a nucleic acid sequence selected from the first extension polynucleotide; and (d2) a second extension product-amplification forward primer comprising a nucleic acid sequence selected from the second extension polynucleotide. The kit for detecting single nucleotide polymorphism may further comprise (d3) an extension product-amplification forward primer comprising a consensus sequence from the first extension polynucleotide and the second extension polynucleotide.

In the kit for detecting single nucleotide polymorphism, the single nucleotide polymorphism may be wild-type or mutant.

The extension polynucleotide may be 20 to 200 nt, 30 to 150 nt, or 40 to 100 nt in length.

In the kit for detecting single nucleotide polymorphism, the first allele may be wild-type, and the second allele may be mutant.

Preferably, the kit for detecting single nucleotide polymorphism in the present invention comprises:
a1) a wild-type allele-specific forward primer comprising: a 5'-end first tag oligonucleotide which does not hybridize to a target nucleic acid under PCR conditions; and a wild-type allele-specific oligonucleotide which is capable of hybridizing specifically to the target nucleic acid and has at its 3'-end, a nucleotide specific for a wild-type allele of the single nucleotide polymorphism, to generate a first PCR product;
a2') a mutant allele-specific forward primer comprising: a 5'-end second tag oligonucleotide which does not hybridize to the target nucleic acid and the 5'-end first tag oligonucleotide under PCR conditions; and a mutant allele-specific oligonucleotide which is capable of hybridizing specifically to the target nucleic acid and has at its 3'-end, a nucleotide specific for a mutant allele of the single nucleotide polymorphism, to generate a second PCR product, or optionally
a2") a mutant allele-specific forward primer comprising a mutant allele-specific oligonucleotide which is capable of hybridizing specifically to the target nucleic acid and has at its 3'-end, a nucleotide specific for the mutant allele of the single nucleotide polymorphism, to generate a second PCR product;
b) a universal reverse primer for amplifying the target nucleic acid;
c1) a first extension primer hybridizing to the complementary of the 5'-end first tag oligonucleotide comprising:
   c11) a 5'-end first extension polynucleotide which does not hybridize to any of the target nucleic acid, the wild-type allele-specific forward primer, the mutant allele-specific forward primer and the reverse primer under PCR conditions, wherein the first extension polynucleotide is 20 to 200 nt and provided for extending the length oft he first PCR product; and
   c12) a 3'-end first tag oligonucleotide which hybridizes to the complementary of the 5'-end first tag oligonucleotide of the first PCR product; c2) a second extension primer hybridizing to the complementary of the 5'-end second tag oligonucleotide, comprising:
      c21) a 5'-end second extension polynucleotide which does not hybridize to any of the target nucleic acid, the wild-type allele-specific forward primer, the mutant allele-specific forward primer and the reverse primer under PCR conditions, the second extension polynucleotide having a length which is distinguishable from that of the first extension polynucleotide in gel electrophoresis, and wherein the second extension polynucleotide is provided for extending the length of the second PCR product; and
      c22) a 3'-end second tag oligonucleotide which hybridizes to the complementary of the 5'-end second tag oligonucleotide of the second PCR product.

The kit for detecting single nucleotide polymorphism may further comprise: d1) a first extension product-amplification forward primer comprising a nucleic acid sequence selected from the first extension polynucleotide; and d2) a second extension product-amplification forward primer comprising a nucleic acid sequence selected from the second extension polynucleotide.

The first extension polynucleotide and the second extension polynucleotide may have different lengths such that the sizes thereof after electrophoresis can be distinguished from each other. For example, when the first extension polynucleotide is 20 to 50 nt in length, the second extension polynucleotide may be 70 to 200 nt in length.

Preferably, the kit for detecting single nucleotide polymorphism in the present invention comprises:
a1) a wild-type allele-specific forward primer comprising: a 5'-end first tag oligonucleotide which does not hybridize to a target nucleic acid under PCR conditions; and a wild-type allele-specific oligonucleotide which is capable of hybridizing specifically to the target nucleic acid and has at its 3'-end, a nucleotide specific for a wild-type allele of the single nucleotide polymorphism, to generate a first PCR product;
a2') a mutant allele-specific forward primer comprising: a 5'-end second tag oligonucleotide which does not hybridize to the target nucleic acid and the 5'-end first tag oligonucleotide under PCR conditions; and a mutant allele-specific oligonucleotide which is capable of hybridizing specifically to the target nucleic acid and has at its 3'-end, a nucleotide specific for a mutant allele of the single nucleotide polymorphism, to generate a second PCR product, or optionally
a2") a mutant allele-specific forward primer comprising a mutant allele-specific oligonucleotide which is capable of hybridizing specifically to the target nucleic acid and has at its 3'-end, a nucleotide specific for the mutant allele of the single nucleotide polymorphism, to generate a second PCR product;
b) a universal reverse primer for amplifying the target nucleic acid;
c1) a first extension primer hybridizing to the complementary of the 5'-end first tag oligonucleotide comprising:
   c11) a 5'-end first extension polynucleotide which does not hybridize to any of the target nucleic acid, the wild-type allele-specific forward primer, the mutant allele-specific forward primer and the reverse primer under PCR conditions, wherein the first extension primer is 20 to 200 nt and provided for extending the length oft he first PCR product; and
   c12) a 3'-end first tag oligonucleotide which hybridizes tot he complementary of the 5'-end first tag oligonucleotide of the first PCR product;
c2) a second extension primer hybridizing to the complementary of the 5'-end second tag oligonucleotide, comprising:
   c21) a 5'-end second extension polynucleotide which does not hybridize to any of the target nucleic acid, the wild-type allele-specific forward primer, the mutant allele-specific forward primer and the reverse primer under PCR conditions, the second extension polynucleotide having a length which is distinguishable from that of the first extension polynucleotide in gel electrophoresis, and wherein the second extension polynucleotide is provided for extending the length of the second PCR product; and
   c22) a 3'-end second tag oligonucleotide which hybridizes to the complementary of the 5'-end second tag oligonucleotide of the second PCR product; and
d) an extension product-amplification forward primer comprising a consensus sequence from the first extension polynucleotide and the second extension polynucleotide.

The kit for detecting single nucleotide polymorphism may further comprise a reaction buffer, a dNTP mixture and a thermally stable DNA polymerase.

In still another aspect of the present invention, there is provided a first method for detecting single nucleotide polymorphism, comprising:
(i) adding, to a reaction solution comprising a target nucleic acid sample to be amplified, a dNTP mixture, a thermally stable DNA polymerase and a reaction buffer,
   (a1) a single nucleotide polymorphic first allele-specific forward primer comprising:
      a 5'-end first tag oligonucleotide which does not hybridize to a target nucleic acid under PCR conditions; and
      a single nucleotide polymorphic first allele-specific oligonucleotide which is capable of hybridizing specifically to the target nucleic acid and has at its 3'-end, a nucleotide specific for the first allele of the single nucleotide polymorphism, to generate a first PCR product;
   (a2') a single nucleotide polymorphic second allele-specific forward primer comprising: a 5'-end second tag oligonucleotide which does not hybridize to the target nucleic acid and the 5'-end first tag oligonucleotide under PCR conditions; and a single nucleotide polymorphic second allele-specific oligonucleotide which is capable of hybridizing specifically to the target nucleic acid and has at its 3'-end, a nucleotide specific for the second allele of the single nucleotide polymorphism, to generate a second PCR product, or optionally
   (a2") a single nucleotide polymorphic second allele-specific forward primer comprising a single nucleotide polymorphic second allele-specific oligonucleotide which is capable of hybridizing specifically to the target nucleic acid and has at its 3'-end, a nucleotide specific for the second allele of the single nucleotide polymorphism, to generate a second PCR product; and
(b) a universal reverse primer for amplifying the target nucleic acid;
(c1) a first extension primer hybridizing to the complementary of the 5'-end first tag oligonucleotide comprising:
   c11) a 5'-end first extension polynucleotide which does not hybridize to any of the target nucleic acid, the single nucleotide polymorphic first allele-specific forward primer and the reverse primer under PCR conditions, wherein the first extension polynucleotide is 20 to 200 nt and provided for extending the length of the first PCR product; and
   c12) a 3'-end first tag oligonucleotide which hybridizes to the complementary of the 5'-end first tag oligonucleotide of the first PCR product; and
(d1) a first extension product-amplification forward primer comprising a nucleic acid sequence selected from the first extension polynucleotide; and
(ii) performing PCR of the reaction solution.

Preferably, the above first method for detecting single nucleotide polymorphism in the present invention further comprises:
(c2) a second extension primer hybridizing to the complementary of the 5'-end second tag oligonucleotide comprising:
   c21) a 5'-end second extension polynucleotide which does not hybridize to any of the target nucleic acid, the single nucleotide polymorphic first allele-specific forward primer, the single nucleotide polymorphic second allele-specific forward primer and the reverse primer under PCR conditions, the second extension polynucleotide having a length which is distinguishable from that of the first extension polynucleotide in gel electrophoresis, and wherein the second extension polynucleotide is provided for extending the length of the second PCR product; and
   c22) a 3'-end second tag oligonucleotide which hybridizes to the complementary of the 5'-end second tag oligonucleotide of the second PCR product; and
(d2) a second extension product-amplification forward primer comprising a nucleic acid sequence selected from the second extension polynucleotide.

In the method for detecting single nucleotide polymorphism, the step of performing the PCR may be performed by a plurality of reactions with an annealing temperature gradient in order to establish optimal hybridization conditions.

In still another aspect of the present invention, there is provided a second method for detecting single nucleotide polymorphism, comprising:
(i) performing allele-specific PCR of a target nucleic acid using:
   (a1) a single nucleotide polymorphic first allele-specific forward primer comprising:
      a 5'-end first tag oligonucleotide which does not hybridize to a target nucleic acid under PCR conditions; and
      a single nucleotide polymorphic first allele-specific oligonucleotide which is capable of hybridizing specifically to the target nucleic acid and has at its 3'-end, a nucleotide specific for the first allele of the single nucleotide polymorphism, to generate a first PCR product;
   (a2') a single nucleotide polymorphic second allele-specific forward primer comprising:
      a 5'-end second tag oligonucleotide which does not hybridize to the target nucleic acid and the 5'-end first tag oligonucleotide under PCR conditions; and
      a single nucleotide polymorphic second allele-specific oligonucleotide which is capable of hybridizing specifically to the target nucleic acid and has at its 3'-end, a nucleotide specific for the second allele of the single nucleotide polymorphism, to generate a second PCR product, or optionally
   (a2") a single nucleotide polymorphic second allele-specific forward primer comprising a single nucleotide polymorphic second allele-specific oligonucleotide which is capable of hybridizing specifically to the target nucleic acid and has at its 3'-end, a nucleotide specific for the second allele of the single nucleotide polymorphism, to generate a second PCR product; and
(b) a universal reverse primer for amplifying the target nucleic acid; and
(ii) performing primer extension of a product of the allele-specific PCR by use of
   (c1) a first extension primer hybridizing to the complementary of the 5'-end first tag oligonucleotide comprising:
   c11) a 5'-end first extension polynucleotide which does not hybridize to any of the target nucleic acid, the single nucleotide polymorphic first allele-specific forward primer and the reverse primer under PCR conditions, wherein the first extension polynucleotide is 20 to 200 nt and provided for extending the length of the first PCR product; and
   c12) a 3'-end first tag oligonucleotide which hybridizes to the complementary of the 5'-end first tag oligonucleotide of the first PCR product.

The method for detecting single nucleotide polymorphism may further comprise iii) performing PCR for amplification of a product of the primer extension, by use of an extension product-amplification forward primer, which comprises a nucleic acid sequence selected from the extension polynucleotide, and the reverse primer.

Preferably, the above second method for detecting single nucleotide polymorphism in the present invention further addes the following to the solution in step (ii):
(c2) a second extension primer hybridizing to the complementary of the 5'-end second tag oligonucleotide comprising:
c21) a 5'-end second extension polynucleotide which does not hybridize to any of the target nucleic acid, the single nucleotide polymorphic first allele-specific forward primer, the single nucleotide polymorphic second allele-specific forward primer and the reverse primer under PCR conditions, the second extension polynucleotide having a length which is distinguishable from that of the first extension polynucleotide in gel electrophoresis, and wherein the second extension polynucleotide is provided for extending the length of the second PCR product; and
c22) a 3'-end second tag oligonucleotide which hybridizes to the complementary of the 5'-end second tag oligonucleotide of the second PCR product.

The method for detecting single nucleotide polymorphism may further comprise iii) performing PCR for amplification of a product of the primer extension, by use of an extension product-amplification forward primer, which comprises a nucleic acid sequence selected from the extension polynucleotide, preferably by use of the following:
(d1) a first extension product-amplification forward primer which comprises a nucleic acid sequence selected from the first extension polynucleotide, and (d2) a second extension product-amplification forward primer comprising a nucleic acid sequence selected from the second extension polynucleotide; or
(d3) an extension product-amplification forward primer comprising a consensus sequence from the first extension polynucleotide and the second extension polynucleotide; and, and
(b) the reverse primer.

Preferably, the above second method for detecting single nucleotide polymorphism in the present invention comprises:
i) performing single nucleotide polymorphism-specific PCR of a target nucleic acid using:
   a1) a wild-type allele-specific forward primer comprising: a 5'-end first tag oligonucleotide which does not hybridize to the target nucleic acid under PCR conditions; and a wild-type allele-specific oligonucleotide which is capable of hybridizing specifically to the target nucleic acid and has at its 3'-end, a nucleotide specific for a wild-type allele of the single nucleotide polymorphism, to generate a second PCR product; and
   a2') a mutant allele-specific forward primer comprising: a 5'-end second tag oligonucleotide which does not hybridize to the target nucleic acid and the first tag oligonucleotide under PCR conditions; and a mutant allele-specific oligonucleotide which is capable of hybridizing specifically to the target nucleic acid and has at its 3'-end, a nucleotide specific for a mutant allele of the single nucleotide polymorphism, to generate a second PCR product, or optionally
   a2") a mutant allele-specific forward primer comprising a mutant allele-specific oligonucleotide which is capable of hybridizing specifically to the target nucleic acid and has at its 3'-end, a nucleotide specific for the mutant allele of the single nucleotide polymorphism, to generate a second PCR product; and
   b) a universal reverse primer for amplifying the target nucleic acid; and
ii) performing primer extension of a product of the single nucleotide polymorphism-specific PCR by use of:
   c1) a first extension primer hybridizing to the complementary of the 5'-end first tag oligonucleotide comprising:
      c11) a 5'-end first extension polynucleotide which does not hybridize to any of the target nucleic acid, the wild-type allele-specific forward primer, the mutant allele-specific forward primer and the reverse primer under PCR conditions, wherein the first extension polynucleotide is 20 to 200 nt and provided for extending the length of the first PCR product; and
      c12) a 3'-end first tag oligonucleotide which hybridizes to the complementary of the 5'-end first tag oligonucleotide of the first PCR product; and
   c2) a second extension primer hybridizing to the complementary of the 5'-end second tag oligonucleotide comprising:
      c21) a 5'-end second extension polynucleotide which does not hybridize to any of the target nucleic acid, the wild-type allele-specific forward primer, the mutant allele-specific forward primer and the reverse primer under PCR conditions, the second extension polynucleotide having a length which is distinguishable from that of the first extension polynucleotide in gel electrophoresis, and wherein the second extension polynucleotide is provided for extending the length of the second PCR product; and
      c22) a 3'-end second tag oligonucleotide which hybridizes to the complementary of the 5'-end second tag oligonucleotide of the second PCR product.

The method for detecting single nucleotide polymorphism may further comprise iii) performing PCR for amplification of a product of the primer extension, by use of a first extension product-amplification forward primer which comprises a nucleic acid sequence selected from the first extension polynucleotide, a second extension product-amplification forward primer which comprises a nucleic acid sequence selected from the second extension polynucleotide, and the reverse primer.

In the method for detecting single nucleotide polymorphism, i) and ii) may be performed by a single step in a single reactor, and i) to iii) may be performed by a single step in a single reactor.

In the method for detecting single nucleotide polymorphism, the single nucleotide polymorphism-specific PCR may be performed by a plurality of reactions with an annealing temperature gradient in order to establish optimal hybridization conditions.

The method for detecting single nucleotide polymorphism may further comprise loading the product of the primer extension or the product of PCR amplification of the primer extension product on gel, subjecting the loaded product to electrophoresis, and then analyzing the band pattern of the product.

Alternatively, the PCR for amplification of the primer extension product may be performed by conventional PCR or real-time PCR. The real-time PCR may be performed using TaqMan probes which bind specifically to the first extension polynucleotide and the second extension polynucleotide, respectively, in addition to a pair of primers, or may be performed using a fluorescent interchelate such as SyBr Green. In this case, the extension product may be specifically detected depending on the kind of fluorescence.

In the method, the PCR for amplification of the primer extension product may be performed by a plurality of reactions with an annealing temperature gradient in order to establish optimal hybridization conditions.

Unless otherwise specified herein, a nucleic acid molecule composed of a plurality of oligonucleotides and/or polynucleotides is read from the 5'-end to the 3'-end.

Hereinafter, a kit and method according to one embodiment of the present invention will be described in further detail with reference to the accompanying drawings.

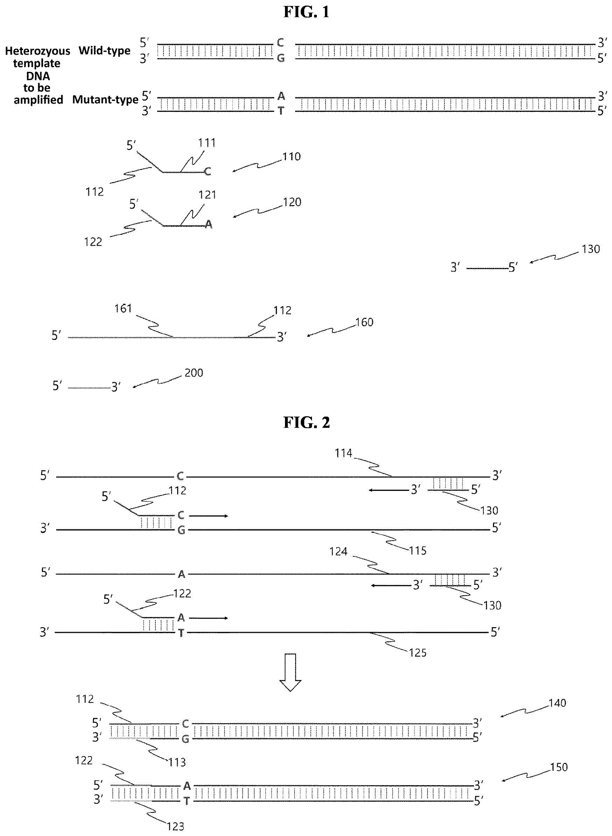

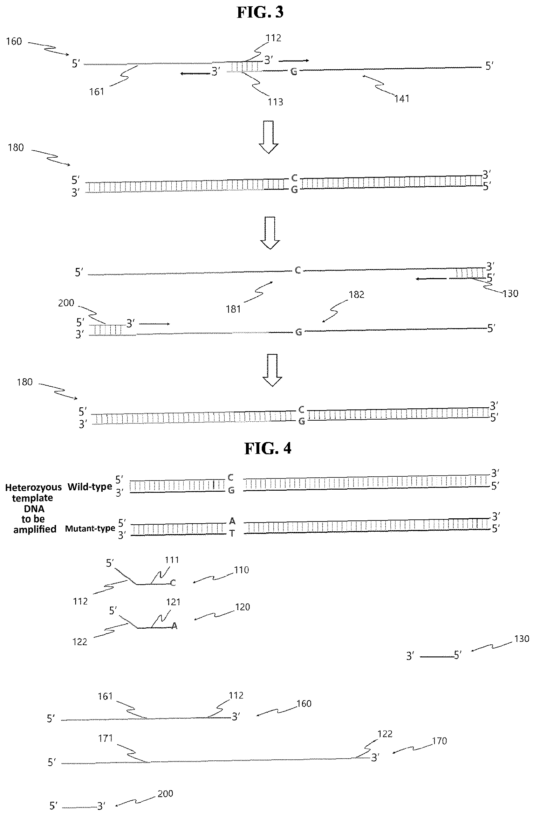

FIG. 1 is a schematic view illustrating a single nucleotide polymorphism to be detected using a single-nucleotide polymorphism detection kit according to an embodiment of the present invention, and various primers for amplifying the same; FIG. 2 is a schematic view illustrating an allele-specific amplification reaction which is performed using a single-nucleotide polymorphism detection kit according to an embodiment of the present invention; and FIG. 3 is a schematic view illustrating an allele-specific asymmetric primer extension reaction and a reaction for amplification of a product of the allele-specific asymmetric primer extension reaction. As shown in FIG. 1, it is possible to conceive of a heterozygote in which both a wild-type allele and a mutant allele exist in a specific single-nucleotide polymorphism site of a specific gene in the genome. As illustrated in FIG. 1, when a C/G base pair is present in the wild-type allele and a C > A (G > T on the side of a complementary strand) mutation is present in the mutant allele, the presence or absence of SNP can be detected by real-time PCR performed using a conventional allele-specific PCR method, primers and an allele-specific fluorescent probe. This method is cost-ineffective, because it uses an expensive fluorescent probe and an expensive real-time PCR system. On the other hand, one embodiment of the present invention is similar to conventional allele-specific PCR (AS-PCR) in that it uses allele-specific primers 110 and 120. However, in one embodiment of the present invention, specific identification tags 112 and 122 that are not present in a target nucleic acid to be amplified are attached to the 5'-end of the allele-specific primers, whereby the presence or absence and type of SNP may be detected without having to use a fluorescent probe or real-time PCR. The 3'-end of the wild-type allele-specific forward primer 110 and the 3'-end of the mutant allele-specific forward primer 120 are the wild-type and mutant nucleotides present in the single-nucleotide polymorphism, respectively. Thus, the wild-type allele-specific forward primer 110 can selectively amplify only the wild-type allele, and the mutant allele-specific forward primer 120 can selectively only the mutant allele (FIG. 2). In this case, a universal reverse primer 130 enables amplification of both the wild-type allele and the mutant allele. Meanwhile, in conventional AS-PCR, PCR is performed by a single reaction only using a wild-type allele-specific primer or a mutant allele-specific primer, and for this reason, PCR reactions should be performed separately to distinguish whether the sample has a wild-type single nucleotide polymorphism or a mutant single nucleotide polymorphism. According to the conventional AS-PCR, it is impossible to confirm whether the single nucleotide polymorphic site is homozygous or heterozygous by a single reaction. As shown in FIGS. 2 and 3, the kit according to the embodiment of the present invention may further comprise an extension primer 160 having a tag oligonucleotide at its 3'-end and an extension oligonucleotide at its 5'-end. The tag oligonucleotide is the identification nucleic acid sequence of any one of the two allele-specific forward primers 110 and 120. In FIG.1, the tag oligonucleotide 112 is the identification nucleic acid sequence of the wild-type allele-specific primer 110 at the 5'-end. The tag oligonucleotide is not complementary to a genomic DNA including a target nucleic acid to be amplified and to a universal reverse primer. The extension oligonucleotide 161 is provided for extending the length of the PCR product amplified by AS-PCR. When primer extension is performed after complementary binding between the tag oligonucleotide of the extension primer 160 and the tag oligonucleotide-complementary sequence of the antisense strand 141 of a wild-type allele amplification product among allele-specific PCR reaction products, allele-specific bidirectional primer extension (ASBPE) is performed (see top of FIG. 3). After the allele-specific bidirectional primer extension is completed, the extension product may be immediately confirmed by agarose gel electrophoresis. When the extension product is amplified using an extension product amplification forward primer 200 selected from the nucleic acid sequence at the 5'-end of the extension oligonucleotide 161 and using the reverse primer used for the allele-specific amplification, an allele-specific extension product can be more reliably detected through amplification of the ASBPE product. In the case of detection by ASBPE reaction alone and the case of detection by ASBPE reaction and extension product amplification, optimal detection conditions can be established by suitably controlling the concentrations of primers used.

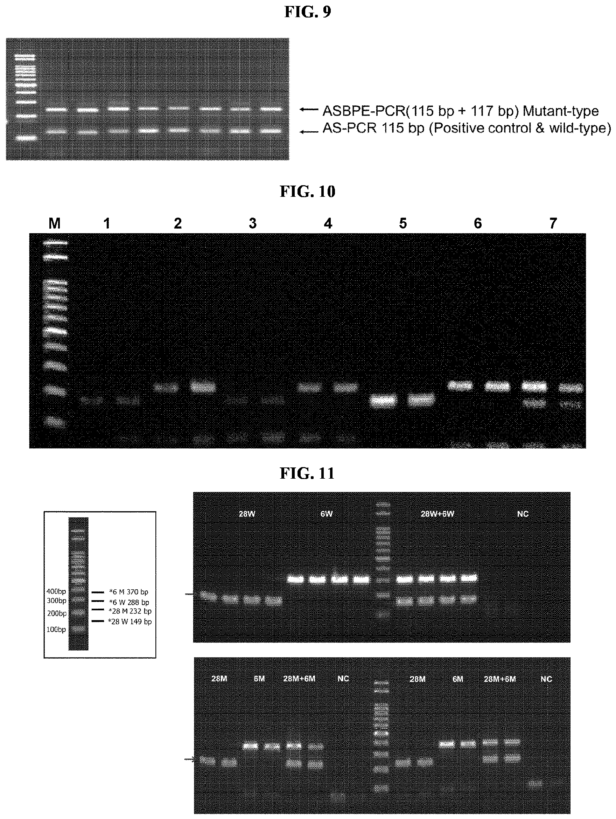

FIG. 9 shows the experimental results obtained by specifically amplifying a heterozygous single-nucleotide polymorphic site according to the method of the embodiment of the present invention as shown in FIGS. 1 to 3. As shown in FIG. 9, the wild-type allele was amplified by allele-specific PCR (AS-PCR) (115 bp), and the mutant allele was amplified by allele-specific bidirectional primer extension PCR of the present invention (115 + 117 bp).

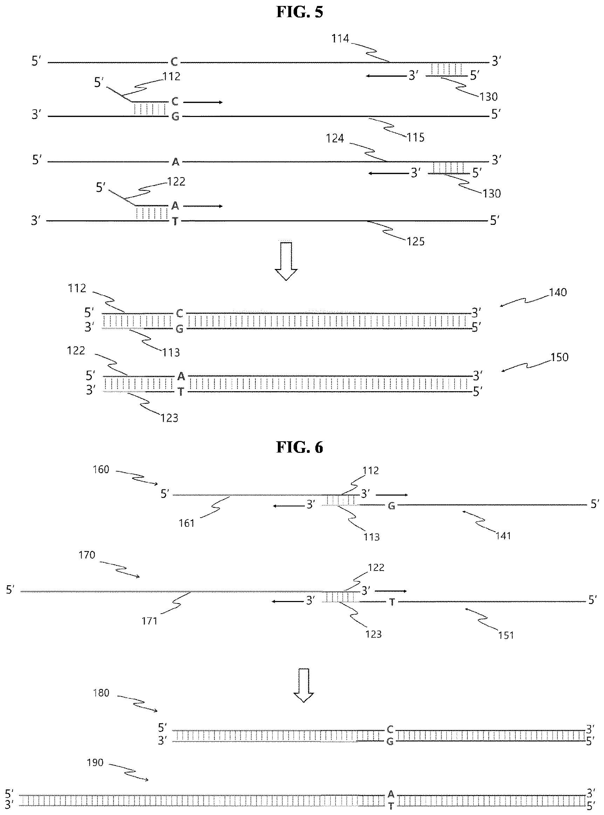

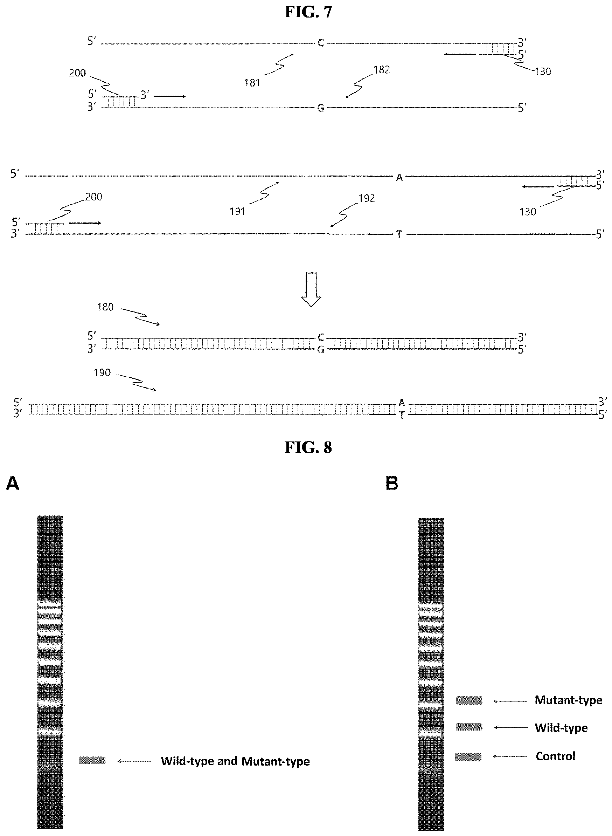

FIGS. 4 to 7 illustrate another embodiment of the present invention. FIG. 4 differs from FIG. 1 in that an extension primer is applied not only to a wild-type allele, but also to a mutant allele, and thus two extension primers are used. Thus, in the embodiment shown in FIG. 4, both wild-type and mutant alleles are amplified by allele-specific bidirectional primer extension PCR after allele-specific PCR. FIG. 5 is a schematic view illustrating an allele-specific PCR process; FIG. 6 is a schematic view illustrating an allele-specific bidirectional primer extension process; and FIG. 7 is a schematic view illustrating a PCR process for the product of the allele-specific bidirectional primer extension. FIG. 8 schematically shows band patterns expected to be obtained when agarose gel electrophoresis is performed after wild-type and mutant alleles are amplified by AS-PCR and allele-specific bidirectional primer extension PCR according to one embodiment of the present invention.

As shown in FIG. 4, the 5'-end of a wild-type allele-specific forward primer 110 and the 5'-end of a mutant allele-specific forward primer 120 are nucleic acid sequences not present in the target nucleic acid to be amplified, and serve as specific tags for a wild-type allele-specific amplification product and a mutant allele-specific amplification product, respectively. For convenience of explanation, the tag included in the wild-type allele-specific forward primer is defined as a first tag oligonucleotide 112, and the tag included in the mutant allele-specific forward primer is defined as a second tag oligonucleotide 122. If there is a plurality of mutations in the single-nucleotide polymorphic site of interest, a third tag oligonucleotide (not shown) and/or a fourth tag oligonucleotide (not shown) may be added to the forward primers. For convenience of explanation and distinction, a mutant allele-specific forward primer comprising the third tag oligonucleotide is defined as a second mutant allele-specific forward primer (not shown), and a mutant allele-specific forward primer comprising the fourth tag nucleotide is defined as a third mutant allele-specific forward primer (not shown). As shown in FIG. 5, a wild-type allele amplification product 140 and a mutant allele amplification product 150 are produced by PCR reaction using the wild-type allele-specific forward primer 110 and the mutant allele-specific forward primer 120, which comprise the first tag oligonucleotide 112 and the second tag oligonucleotide 122, respectively, and the universal reverse primer 130. The two PCR products in this step are not distinguished from each other, because they have the same size and their bands on electrophoresis gel overlap each other. The first tag oligonucleotide 112 and the second tag oligonucleotide 122 are, respectively, used as the 3'-end sequence of a first extension primer and the 3'-end sequence of a second extension primer, which are used for primer extension. Thus, as shown in FIG. 6, the antisense strand 141 of the wild-type amplification product, made single-stranded by denaturation, hybridizes specifically to the first tag oligonucleotide 112 located at the 3'-end of the first extension primer 160, and the antisense strand 151 of the mutant allele amplification product hybridizes specifically to the second tag oligonucleotide 122 located at the 3'-end of the second extension primer 170, thereby producing a first extension product 180 and a second extension product 190, respectively. In this case, since the first extension primer 160 and the second extension primer 170 have different lengths, the first extension product 180 and the second extension product 190 can be distinguished from each other by analyzing their band patterns using gel electrophoresis. The present inventor named this allele-specific primer extension as allele specific bidirectional primer extension (ASBPE). Single nucleotide polymorphism can be detected only by the ASBPE. However, when the concentration of a template nucleic acid molecule is low, it may be difficult to detect an allele-specific nucleic acid molecule only by primer extension. In this case, as shown in FIG. 4, more reliable detection of single nucleotide polymorphism can be achieved by amplifying the first extension product and the second extension product using an extension product amplification forward primer 200, constructed using a specific nucleic sequence selected from the first extension primer and the second extension primer, and the above-described universal reverse primer 130. The extension product amplification forward primer 200 that is used in this case may be constructed using a sequence present commonly in the first extension primer and the second extension primer, and may also be constructed using specific nucleic acid sequences that are different from each other. In FIGS. 4 and 7 which illustrate one embodiment of the present invention, there is shown only the extension product amplification forward primer 200 constructed using a sequence present commonly in the first extension primer 160 and the second extension primer 170.

FIG. 10 shows the results of an actual experiment performed according to the above-described embodiment. As shown in FIG. 10, allele-specific bidirectional primer extension PCR of the present invention was performed on homozygous or heterozygous UGT1A1^{∗}28. As a result, only a wild-type allele (181 bp) was detected by the wild-type allele-specific forward primer and its extension primer, and only a mutant allele (231 bp) was detected by the mutant allele-specific forward primer and its extension primer. In addition, in heterozygous UGT1A1^{∗}28, the two alleles were all detected.

FIG. 11 shows the results of multiplex PCR performed to simultaneously detect a plurality of alleles according to an example of the present invention. UGT1A1^{∗}6 and UGT1A1^{∗}28 were amplified by allele-specific bidirectional primer extension PCR according to an example of the present invention. As a result, the two alleles could all be detected while they were accurately distinguished between the wild type and the mutant.

### ADVANTAGEOUS EFFECTS

According to an embodiment of the present invention, it is possible to detect the presence or absence and kind of single nucleotide polymorphism in a certain gene in an accurate and rapid manner. Thus, the kit and method for detecting single nucleotide polymorphism according to the embodiment of the present invention may be very advantageously used in companion diagnostics to provide important information on the diagnosis of various diseases including cancer, particularly the presence or absence of responsiveness to a specific drug and the choice of an alternative drug.

### BRIEF DESCRIPTION OF DRAWINGS

FIG. 1 schematically illustrates a single-nucleotide polymorphism to be amplified by a single-nucleotide polymorphism detection kit according to an embodiment of the present invention, and various primers for amplifying the single-nucleotide polymorphism.
FIG. 2 schematically illustrates an allele-specific nucleic acid amplification reaction which is performed by a single-nucleotide polymorphism detection kit according to an embodiment of the present invention.
FIG. 3 schematically illustrates an allele-specific asymmetric primer extension reaction according to an embodiment of the present invention, and amplification of a product of the allele-specific asymmetric primer extension reaction.
FIG. 4 schematically illustrates a single-nucleotide polymorphism to be amplified by a single-nucleotide polymorphism detection kit according to an embodiment of the present invention, and various primers for amplifying the single-nucleotide polymorphism.
FIG. 5 schematically illustrates an allele-specific nucleic acid amplification reaction which is performed by a single-nucleotide polymorphism detection kit according to an embodiment of the present invention.
FIG. 6 schematically illustrates an allele-specific bidirectional primer extension reaction according to an embodiment of the present invention, which is performed using an allele-specific extension primer after the allele-specific nucleic acid amplification reaction shown in FIG. 5.
FIG. 7 schematically illustrates a nucleic acid amplification reaction in which a product of the allele-specific bidirectional primer extension reaction is amplified by PCR.
FIG. 8 is a schematic view illustrating the results of agarose gel electrophoresis for a product of conventional allele-specific PCR (AS-PCR) and a product of allele-specific bidirectional primer amplification according to an embodiment of the present invention, or amplification products obtained by further amplifying the above products.
FIG. 9 is a photograph showing the results of allele-specific PCR performed according to an embodiment of the present invention.
FIG. 10 is a photograph showing the results of allele-specific bidirectional primer extension PCR performed according to an embodiment of the present invention.
FIG. 11 is a photograph showing the results of the multiplex allele-specific PCR (top) and multiplex allele-specific bidirectional primer extension PCR (bottom) performed according to an embodiment of the present invention.

### Examples

Hereinafter, the present invention will be described with reference to non-limiting examples. It is to be understood, however, that these examples are for illustrative purposes only and are not intended to limit the scope of the present invention.

### Example 1: Allele-Specific Amplification of Target Nucleic Acid Molecule

In the present invention, in order to examine whether the presence or absence and type of single nucleotide polymorphism (SNP) can be determined rapidly by the allele-specific bidirectional primer extension as described above, examination was first performed to determine whether allele-specific PCR would be performed using as a template the BCR-ABL gene known to have SNP.

Specifically, in order to detect SNP (C > T) corresponding to the 318^{th} nucleotide of the template DNA (human c-abl oncogene) represented by SEQ ID NO: 1, a 115 bp amplification product was produced by allele-specific PCR using a C-specific primer represented by SEQ ID NO: 2 and a T-specific primer represented by SEQ ID NO: 3, which are allele-specific primers, and a reverse primer represented by SEQ ID NO: 4. In this case, the two forward primers all had a deliberate mismatch (C > A) introduced to the 3rd nucleotide from the 3'-end. This mismatch is known to allow amplification of allele-specific primers to be more stably performed (Liu et al. Plant Methods 2012, 8:34, 2012). In addition, for optimization of the annealing temperatures of the primers, temperature-gradient PCR was performed while the annealing temperatures of a plurality of reactants were sequentially controlled. The PCR reaction was performed three times. The composition of the PCR reaction solution used and the PCR reaction conditions are shown in Tables 1 and 2, respectively.

**Table 1: Comnosition of allele-SDecific PCR reaction solution**

| PCR Composition | | | | |
|---|---|---|---|---|
| Sample # | | | (µl) | End-Conc. |
| 5X PCR Buffer | | | 10 | 1X |
| Go tag DNA polymerase | | | 0.25 | 1.25 U |
| dNTP Mix (10 mM) | | | 1 | 200 µM |
| Template | C, T | | 1 | 10⁵ copies |
| Primer | F | | 1 | 100 nM |
| | R | | 1 | 100 nM |
| DI water | | | Balance | |
| Total Vol. | | | 50 | |

**Table 2: Conditions of allele-specific PCR reaction**

| Temperature | Time | |
|---|---|---|
| 95°C | 2 min | |
| 95°C | 30 sec | 35cycle |
| 53, 55, 57, 59, 61 or 63°C | 30 sec | |
| 72°C | 30 sec | |
| 72°C | 5 min | |
| 4°C | ∞ | |

The results of the temperature-gradient AS-PCR indicated that the C-specific forward primer and the T-specific forward primer formed wild-type and mutant allele-specific amplification products, respectively (see FIG. 9). Thus, it was shown that the allele-specific primers according to the embodiment of the present invention operated properly. Meanwhile, in order to examine the optimal annealing temperature, temperature-gradient PCR was performed using various annealing temperatures. As a result, it could be seen that an annealing temperature of about 57°C was optimal for both the wild-type and mutant alleles.

### Example 2: Construction of Extension Primer

The present inventor prepared an extension primer to be used in allele-specific bidirectional primer extension following allele-specific PCR. Specifically, amplification was performed using the pBR322 plasmid (Promega, USA) represented by SEQ ID NO: 5, which has a low sequence homology with human genomic DNA, as a template and using a forward primer represented by SEQ ID NO: 6 and a reverse primer represented by SEQ ID NO: 7, thereby producing a 117 bp amplification product. Here, the forward primer was composed of the homologous sequence present in the pBR322 plasma and a second tag complementary oligonucleotide (SEQ ID NO: 8), attached to the 5'-end of the homologous sequence and complementary to a second tag oligonucleotide to be described below.

### Example 3: Construction of Tag-Containing Primer and ASBPE Reaction Using the Same

In the present invention, a tag oligonucleotide having a specific nucleic acid sequence, which has a low homology with any sequence in the human genome and thus shows no false-positive results, was designed in the 5'-end of the forward primer in AS-PCR, and allele-specific PCR was performed using the forward primer having the designed tag oligonucleotide. The tag oligonucleotide is also known as the ZIP code sequence in the technical field to which the present invention pertains. The ZIP code sequence is a nucleotide oligomer having a sequence not present in the nucleotide sequence of genomic DNA, and is a sequence designed so as not to hybridize non-specifically to genomic DNA. Any person skilled in the technical field to which the present invention pertains will easily understand that the primer may be modified using various code sequences known in the art, in addition to the ZIP code sequences used in the Examples below (see Slentz-Kesler et al., Genome Res., 10: 549-557, 2010).

Specifically, in the present invention, a mutant allele-specific primer (SEQ ID NO: 9) was constructed by attaching, to the 5'-end of the mutant allele-specific forward primer (SEQ ID NO: 3) used in Example 1, a specific tag oligonucleotide (second tag oligonucleotide) represented by SEQ ID NO: 10 which is complementary to the second tag complementary oligonucleotide represented by SEQ ID NO: 8 and is capable of identifying the mutant allele. Using the wild-type allele-specific forward primer constructed in Example 1, the mutant allele-specific primer constructed as described above, the reverse primer of SEQ ID NO: 7 used for construction of the extension primer in Example 2 (the reverse primer acted as a forward primer for amplification of an extension product in this PCR), and the universal reverse primer used in Example 1, PCR was performed in the same manner as described in Example 1, except that the annealing temperature was set to 57°C. As a result, as shown in FIG. 9, the following two fragments were detected: a 115 bp fragment amplified by allele-specific PCR using the wild-type allele-specific forward primer and the universal reverse primer; and a 232 bp fragment produced by the allele-specific bidirectional primer extension according to the embodiment of the present invention.

### Example 4: SNP Analysis of UGTIAI Gene

In the present invention, the genotype of UGT1A1^{∗}28, which is the SNP of UDP glucuronosyltransferase family 1 member A1 (UGT1A1) known to be correlated with the side effects of the anticancer drug Irinotecan, was analyzed using an ASBPE kit according to an embodiment of the present invention.

Specifically, using a wild-type allele-specific forward primer (SEQ ID NO: 11) and a mutant allele-specific forward primer (SEQ OD NO: 12) for UGT1A1^{∗}28 and a reverse primer (SEQ ID NO: 13) for UGT1A1^{∗}28, allele-specific PCR was performed.

Next, primer extension was performed using a wild-type allele-specific extension primer (SEQ ID NO: 14) for the wild-type allele and a mutant allele-specific extension primer (SEQ ID NO: 15) for the mutant allele.

Finally, amplification of the wild-type allele extension product and the mutant allele extension product was performed using a universal extension product amplification forward primer (SEQ ID NO: 16) and the reverse primer (SEQ ID NO: 13).

However, the above-described three reactions were not sequentially performed, but were performed in a single step in a single reaction tube under the following conditions.

**Table 3: Allele-specific ASBPE-PCR reaction conditions**

| | | |
|---|---|---|
| 95°C | 10 min | Number of cycles |
| 95°C | 30 sec | 35 cycles |
| 58°C | 30 sec | |
| 72°C | 30 sec | |
| 72°C | 5 min | |
| 4°C | ∞ | |

As a result, as shown in FIG. 10, the mutant allele could be amplified using the ASBPE kit according to the embodiment of the present invention, and the wild-type allele could be amplified using AS-PCR while the two alleles could be clearly distinguished from each other.

### Example 5: Multiplex Analysis of SNP of UGT1A1 Gene

In addition, in the present invention, in order to examine whether multiplex analysis capable of detecting the genotypes of the two alleles at the same time by the ASBPE kit according to the embodiment of the present invention is possible, allele-specific PCR was performed in a single or multiplex manner using the wild-type genomic DNAs of UGT1A1^{∗}6 and UGT1A1^{∗}28 alone or as a mixture, and PCR using the ASBPE kit according to the embodiment of the present invention was performed in multiplex using the mutant genomic DNAs of UGT1A1^{∗}6 and UGT1A1^{∗}28 alone or as a mixture.

Specifically, UGT1A1^{∗}6 wild-type genomic DNA and UGT1A1^{∗}28 wild-type genomic DNA were prepared alone or as a mixture, and then using the genomic DNA as a template, allele-specific PCR was performed using each of wild-type allele-specific forward primers (SEQ ID NO: 17 and SEQ ID NO: 11) and each of reverse primers (SEQ ID NO: 18 and SEQ ID NO: 13) under the conditions shown in Table 3 above. As a result, as shown in the top of FIG. 11, UGT1A1^{∗}6 wild-type genomic DNA and UGT1A1^{∗}28 wild-type genomic DNA could also be specifically amplified by AS-PCR. In addition, in the present invention, UGT1A1^{∗}6 mutant-type genomic DNA and UGT1A1^{∗}28 mutant genomic DNA were prepared alone or as a mixture, and then using the genomic DNA as a template, allele-specific bidirectional primer extension PCR (ASBPE-PCR) was performed using each of mutant allele-specific forward primers (SEQ ID NO: 19 and SEQ ID NO: 12), a universal reverse primer (SEQ ID NO: 13), each of UGT1A1^{∗}6 mutant-specific and UGT1A1^{∗}28 mutant-specific extension primers (SEQ ID NO: 20 and SEQ ID NO: 15), and an extension product-amplification forward primer (SEQ ID NO: 16) comprising a consensus sequence present at the 5'-end of the mutant-specific extension primers for each of UGT1A1^{∗}6 and UGT1A1^{∗}28, under the conditions shown in Table 3.

As a result, as shown in FIG. 11, the allele-specific bidirectional primer extension PCR kit according to the embodiment of the present invention could clearly detect the mutant allele of each of UGT1A1^{∗}6 and UGT1A1^{∗}28 in both single and multiplex formats. Furthermore, these results were obtained through a single PCR process composed of allele-specific PCR, allele-specific bidirectional primer extension, and PCR amplification of the extension product, and these results demonstrated that the method and kit according to the embodiment of the present invention may be used to distinguish between a wild-type allele and a mutant allele in a multiplex format within the same time period as conventional AS-PCR and may also be used to detect a variety of SNPs at the same time.

Thus, the allele-specific bidirectional primer extension according to the embodiment of the present invention may be used to accurately and effectively determine the presence or absence and kind of SNP at a specific position in a specific target nucleic acid molecule. Furthermore, the method according to the embodiment of the present invention is a very cost-effective in that it may be performed by a single reaction in a single tube and in that it may be performed in an easy and inexpensive manner by analyzing band patterns after simple gel electrophoresis without having to use an expensive device.

As described above, the method and kit according to the embodiment of the present invention may be used to detect SNP in various animals and plants, particularly humans, and may also be used to diagnose diseases and predict drug responsiveness on the basis of the detection results.
## Claims

1. A kit for detecting single nucleotide polymorphism, comprising:
(a1) a single nucleotide polymorphic first allele-specific forward primer (110) comprising:
a 5'-end first tag oligonucleotide (112) which does not hybridize to a target nucleic acid under PCR conditions; and
a single nucleotide polymorphic first allele-specific oligonucleotide (111) which is capable of hybridizing specifically to the target nucleic acid and has at its 3'-end, a nucleotide specific for the first allele of the single nucleotide polymorphism, to generate a first PCR product (140);
(a2') a single nucleotide polymorphic second allele-specific forward primer (120) comprising:
a 5'-end second tag oligonucleotide (122) which does not hybridize to the target nucleic acid and the 5'-end first tag oligonucleotide (112) under PCR conditions; and
a single nucleotide polymorphic second allele-specific oligonucleotide (121) which is capable of hybridizing specifically to the target nucleic acid and has at its 3'-end, a nucleotide specific for the second allele of the single nucleotide polymorphism, to generate a PCR second product (150), or optionally
(a2") a single nucleotide polymorphic second allele-specific forward primer (120) comprising a single nucleotide polymorphic second allele-specific oligonucleotide (121) which is capable of hybridizing specifically to the target nucleic acid and has at its 3'-end, a nucleotide specific for the second allele of the single nucleotide polymorphism, to generate a PCR second product (150); and
(b) a universal reverse primer (130) for amplifying the target nucleic acid; and
(c1) a first extension primer (160) hybridizing to the complementary (113) of the 5'-end first tag oligonucleotide (112) comprising:
c11) a 5'-end first extension polynucleotide which does not hybridize to any of the target nucleic acid, the single nucleotide polymorphic first allele-specific forward primer (110) and the reverse primer (130) under PCR conditions, wherein the first extension polynucleotide is 20 to 200 nt and provided for extending the length of the first PCR product (140); and
c12) a 3'-end first tag oligonucleotide which hybridizes to the complementary (113) of the 5'-end first tag oligonucleotide (112) of the first PCR product (140).

2. The kit of claim 1, further comprising:
(c2) a second extension primer (170) hybridizing to the complementary (123) of the 5'-end second tag oligonucleotide (122) comprising:
c21) a 5'-end second extension polynucleotide which does not hybridize to any of the target nucleic acid, the single nucleotide polymorphic first allele-specific forward primer (110), the single nucleotide polymorphic second allele-specific forward primer (120) and the reverse primer (130) under PCR conditions, the second extension polynucleotide having a length which is distinguishable from that of the first extension polynucleotide in gel electrophoresis, and wherein the second extension polynucleotide is provided for extending the length of the second PCR product (150); and
c22) a 3'-end second tag oligonucleotide which hybridizes to the complementary (123) of the 5'-end second tag oligonucleotide (122) of the second PCR product (150).

3. The kit of claim 2, further comprising:
(d1) a first extension product-amplification forward primer (200) comprising a nucleic acid sequence selected from the first extension polynucleotide; and
(d2) a second extension product-amplification forward primer (200) comprising a nucleic acid sequence selected from the second extension polynucleotide.

4. The kit of claim 2, further comprising:
(d3) an extension product-amplification forward primer (200) comprising a consensus sequence from the first extension polynucleotide and the second extension polynucleotide.

5. The kit of any one of claims 1 to 4, wherein the first allele is mutant and the second allele is wild-type; or the first allele is wild-type and the second allele is mutant.

6. The kit of any one of claims 1 to 5, further comprising a reaction buffer, a dNTP mixture and a thermally stable DNA polymerase.

7. A method for detecting single nucleotide polymorphism, comprising:
(i) adding, to a reaction solution comprising a target nucleic acid sample to be amplified, a dNTP mixture, a thermally stable DNA polymerase and a reaction buffer,
(a1) a single nucleotide polymorphic first allele-specific forward primer (110) comprising:
a 5'-end first tag oligonucleotide (112) which does not hybridize to a target nucleic acid under PCR conditions; and
a single nucleotide polymorphic first allele-specific oligonucleotide (111) which is capable of hybridizing specifically to the target nucleic acid and has at its 3'-end, a nucleotide specific for the first allele of the single nucleotide polymorphism, to generate a first PCR product (140);
(a2') a single nucleotide polymorphic second allele-specific forward primer (120) comprising: a 5'-end second tag oligonucleotide (122) which does not hybridize to the target nucleic acid and the 5'-end first tag oligonucleotide (112) under PCR conditions; and a single nucleotide polymorphic second allele-specific oligonucleotide (121) which is capable of hybridizing specifically to the target nucleic acid and has at its 3'-end, a nucleotide specific for the second allele of the single nucleotide polymorphism, to generate a second PCR product (150), or optionally
(a2") a single nucleotide polymorphic second allele-specific forward primer (120) comprising a single nucleotide polymorphic second allele-specific oligonucleotide (121) which is capable of hybridizing specifically to the target nucleic acid and has at its 3'-end, a nucleotide specific for the second allele of the single nucleotide polymorphism, to generate a second PCR product (150); and
(b) a universal reverse primer (130) for amplifying the target nucleic acid;
(c1) a first extension primer (160) hybridizing to the complementary (113) of the 5'-end first tag oligonucleotide (112) comprising:
c11) a 5'-end first extension polynucleotide which does not hybridize to any of the target nucleic acid, the single nucleotide polymorphic first allele-specific forward primer (110) and the reverse primer (130) under PCR conditions, wherein the first extension polynucleotide is 20 to 200 nt and provided for extending the length of the first PCR product (140); and
c12) a 3'-end first tag oligonucleotide which hybridizes to the complementary (113) of the 5'-end first tag oligonucleotide (112) of the first PCR product (140); and
(d1) a first extension product-amplification forward primer (200) comprising a nucleic acid sequence selected from the first extension polynucleotide; and
(ii) performing PCR of the reaction solution.

8. The method of claim 7, wherein in (i), further adding to the reaction solution the following:
(c2) a second extension primer (170) hybridizing to the complementary (123) of the 5'-end second tag oligonucleotide (122) comprising:
c21) a 5'-end second extension polynucleotide which does not hybridize to any of the target nucleic acid, the single nucleotide polymorphic first allele-specific forward primer (110), the single nucleotide polymorphic second allele-specific forward primer (120) and the reverse primer (130) under PCR conditions, the second extension polynucleotide having a length which is distinguishable from that of the first extension polynucleotide in gel electrophoresis, and wherein the second extension polynucleotide is provided for extending the length of the second PCR product (150); and
c22) a 3'-end second tag oligonucleotide which hybridizes to the complementary (123) of the 5'-end second tag oligonucleotide (122) of the second PCR product (150); and
(d2) a second extension product-amplification forward primer (200) comprising a nucleic acid sequence selected from the second extension polynucleotide.

9. A method for detecting single nucleotide polymorphism, comprising:
(i) performing allele-specific PCR of a target nucleic acid using:
(a1) a single nucleotide polymorphic first allele-specific forward primer (110) comprising:
a 5'-end first tag oligonucleotide (112) which does not hybridize to a target nucleic acid under PCR conditions; and
a single nucleotide polymorphic first allele-specific oligonucleotide (111) which is capable of hybridizing specifically to the target nucleic acid and has at its 3'-end, a nucleotide specific for the first allele of the single nucleotide polymorphism, to generate a first PCR product (140);
(a2') a single nucleotide polymorphic second allele-specific forward primer (120) comprising:
a 5'-end second tag oligonucleotide (122) which does not hybridize to the target nucleic acid and the 5'-end first tag oligonucleotide (112) under PCR conditions; and
a single nucleotide polymorphic second allele-specific oligonucleotide (121) which is capable of hybridizing specifically to the target nucleic acid and has at its 3'-end, a nucleotide specific for the second allele of the single nucleotide polymorphism, to generate a second PCR product (150), or optionally
(a2") a single nucleotide polymorphic second allele-specific forward primer (120) comprising a single nucleotide polymorphic second allele-specific oligonucleotide (121) which is capable of hybridizing specifically to the target nucleic acid and has at its 3'-end, a nucleotide specific for the second allele of the single nucleotide polymorphism, to generate a second PCR product (150); and
(b) a universal reverse primer (130) for amplifying the target nucleic acid; and
(ii) performing primer extension of a product of the allele-specific PCR by use of (c1) a first extension primer (160) hybridizing to the complementary (113) of the 5'-end first tag oligonucleotide (112) comprising:
c11) a 5'-end first extension polynucleotide which does not hybridize to any of the target nucleic acid, the single nucleotide polymorphic first allele-specific forward primer (110) and the reverse primer (130) under PCR conditions, wherein the first extension polynucleotide is 20 to 200 nt and provided for extending the length of the first PCR product (140); and
c12) a 3'-end first tag oligonucleotide which hybridizes to the complementary (113) of the 5'-end first tag oligonucleotide (112) of the first PCR product (140).

10. The method of claim 9, wherein in (ii), further adding to the solution,
(c2) a second extension primer (170) hybridizing to the complementary (123) of the 5'-end second tag oligonucleotide (122) comprising:
c21) a 5'-end second extension polynucleotide which does not hybridize to any of the target nucleic acid, the single nucleotide polymorphic first allele-specific forward primer (110), the single nucleotide polymorphic second allele-specific forward primer (120) and the reverse primer (130) under PCR conditions, the second extension polynucleotide having a length which is distinguishable from that of the first extension polynucleotide in gel electrophoresis, and wherein the second extension polynucleotide is provided for extending the length of the second PCR product (150); and
c22) a 3'-end second tag oligonucleotide which hybridizes to the complementary (123) of the 5'-end second tag oligonucleotide (122) of the second PCR product (150).

11. The method of claim 10, further comprising:
(iii) performing PCR for amplification of a product of the primer extension, by use of the following:
(d1) a first extension product-amplification forward primer (200) which comprises a nucleic acid sequence selected from the first extension polynucleotide, and
(d2) a second extension product-amplification forward primer (200) comprising a nucleic acid sequence selected from the second extension polynucleotide; or
(d3) an extension product-amplification forward primer (200) comprising a consensus sequence from the first extension polynucleotide and the second extension polynucleotide; and
(b) the reverse primer (130).

12. The method of claim 7 or 8, wherein (i) and (ii) are performed by a single step in a single reactor.

13. The method of claim 11, wherein (i), (ii) and (iii) are performed by a single step in a single reactor.

## Patentansprüche

1. Kit zum Nachweis von Einzelnukleotid-Polymorphismus, umfassend:
(a1) einen Einzelnukleotid-polymorphen erstes Allel-spezifischen Vorwärtsprimer (110), umfassend:
ein erstes 5'-Ende-Tag-Oligonukleotid (112), das unter PCR-Bedingungen nicht an eine Zielnukleinsäure hybridisiert; und
ein Einzelnukleotid-polymorphes erstes Allel-spezifisches Oligonukleotid (111), das spezifisch mit der Zielnukleinsäure hybridisieren kann und an seinem 3 '-Ende ein Nukleotid aufweist, das spezifisch für das erste Allel des Einzelnukleotid-Polymorphismus ist, um ein erstes PCR-Produkt zu erzeugen (140);
(a2') einen Einzelnukleotid-polymorphen zweites Allel-spezifischen Vorwärtsprimer (120), umfassend:
ein zweites 5'-Ende-Tag-Oligonukleotid (122), das unter PCR-Bedingungen nicht mit der Zielnukleinsäure und dem ersten 5'-Ende-Tag-Oligonukleotid (112) hybridisiert; und
ein Einzelnukleotid-polymorphes zweites Allel-spezifisches Oligonukleotid (121), das spezifisch an die Zielnukleinsäure hybridisieren kann und an seinem 3'-Ende ein für das zweite Allel des Einzelnukleotid-Polymorphismus spezifisches Nukleotid aufweist, um ein zweites PCR-Produkt (150) zu erzeugen, oder optional
(a2") einen Einzelnukleotid-polymorphen zweites Allel-spezifischen Vorwärtsprimer (120), umfassend ein Einzelnukleotid-polymorphes zweites Allel-spezifisches Oligonukleotid (121), das spezifisch an die Zielnukleinsäure hybridisieren kann und an seinem 3'-Ende ein für das zweite Allel des Einzelnukleotid-Polymorphismus spezifisches Nukleotid aufweist, um ein zweites PCR-Produkt (150) zu erzeugen; und
(b) einen universellen Rückwärtsprimer (130) zur Amplifikation der Zielnukleinsäure; und (c1) einen ersten Verlängerungsprimer (160), der mit dem Komplementären (113) des ersten 5'-Ende-Tag-Oligonukleotids (112) hybridisiert, umfassend:
c11) ein erstes 5'-Ende-Verlängerungspolynukleotid, das unter PCR-Bedingungen mit keiner der Zielnukleinsäuren, dem Einzelnukleotid-polymorphen erstes Allel-spezifischen Vorwärtsprimer (110) und dem Rückwärtsprimer (130) hybridisiert, wobei das erste Verlängerungspolynukleotid 20 bis 200 nt beträgt und zur Verlängerung des ersten PCR-Produkts (140) vorgesehen ist; und
c12) ein erstes 3'-Ende-Tag-Oligonukleotid, das mit dem Komplementären (113) des ersten 5'-Ende-Tag-Oligonukleotids (112) des ersten PCR-Produkts (140) hybridisiert.

2. Kit nach Anspruch 1, ferner umfassend:
(c2) einen zweiten Verlängerungsprimer (170), der mit dem Komplementären (123) des zweiten 5'-Ende-Tag-Oligonukleotids (122) hybridisiert, umfassend:
c21) ein zweites 5'-Ende-Verlängerungspolynukleotid, das unter PCR-Bedingungen mit keiner der Zielnukleinsäuren, dem Einzelukleotid-polymorphen erstes Allel-spezifischen Vorwärtsprimer (110), dem Einzelnukleotid-polymorphen zweites Allel-spezifischen Vorwärtsprimer (120) und dem Rückwärtsprimer (130) hybridisiert, wobei das zweite Verlängerungspolynukleotid eine Länge aufweist, die von der des ersten Verlängerungspolynukleotids in der Gelelektrophorese unterscheidbar ist, und wobei das zweite Verlängerungspolynucleotid zum Verlängern der Länge des zweiten PCR-Produkts (150) vorgesehen ist; und
c22) ein zweites 3'-Ende-Tag-Oligonukleotid, das mit dem Komplementären (123) des zweiten 5'-Ende-Tag-Oligonukleotids (122) des zweiten PCR-Produkts (150) hybridisiert.

3. Kit nach Anspruch 2, ferner umfassend:
(d1) einen ersten Verlängerungs-Produktamplifikations-Vorwärtsprimer (200), der eine Nukleinsäuresequenz umfasst, die aus dem ersten Verlängerungspolynukleotid ausgewählt ist; und
(d2) einen zweiten Verlängerungs-Produktamplifikations-Vorwärtsprimer (200), umfassend eine Nukleinsäuresequenz, die aus dem zweiten Verlängerungspolynukleotid ausgewählt ist.

4. Kit nach Anspruch 2, ferner umfassend:
(d3) einen Verlängerungs-Produktamplifikations-Vorwärtsprimer (200), umfassend eine Konsensussequenz aus dem ersten Verlängerungspolynukleotid und dem zweiten Verlängerungspolynukleotid.

5. Kit nach einem der Ansprüche 1 bis 4, wobei das erste Allel mutiert ist und das zweite Allel vom Wildtyp ist; oder das erste Allel vom Wildtyp ist und das zweite Allel mutiert ist.

6. Kit nach einem der Ansprüche 1 bis 5, ferner umfassend einen Reaktionspuffer, eine dNTP-Mischung und eine thermisch stabile DNA-Polymerase.

7. Verfahren zum Nachweis von Einzelnukleotid-Polymorphismus, umfassend:
(i) die Zugabe einer dNTP-Mischung, einer thermisch stabilen DNA-Polymerase und eines Reaktionspuffers zu einer Reaktionslösung, die eine zu amplifizierende Zielnukleinsäureprobe umfasst,
(a1) einen Einzelnukleotid-polymorphen erstes Allel-spezifischen Vorwärtsprimer (110), umfassend:
ein erstes 5'-Ende-Tag-Oligonukleotid (112), das unter PCR-Bedingungen nicht an eine Zielnukleinsäure hybridisiert; und
ein Einzelnukleotid-polymorphes erstes Allel-spezifisches Oligonukleotid (111), das spezifisch mit der Zielnukleinsäure hybridisieren kann und an seinem 3 '-Ende ein Nukleotid aufweist, das spezifisch für das erste Allel des Einzelnukleotid-Polymorphismus ist, um ein erstes PCR-Produkt zu erzeugen (140);
(a2') einen Einzelnukleotid-polymorphen zweites Allel-spezifischen Vorwärtsprimer (120), umfassend:
ein zweites 5'-Ende-Tag-Oligonukleotid (122), das unter PCR-Bedingungen nicht mit der Zielnukleinsäure und dem ersten 5'-Ende-Tag-Oligonukleotid (112) hybridisiert; und
ein Einzelnukleotid-polymorphes zweites Allel-spezifisches Oligonukleotid (121), das spezifisch an die Zielnukleinsäure hybridisieren kann und an seinem 3'-Ende ein für das zweite Allel des Einzelnukleotid-Polymorphismus spezifisches Nukleotid aufweist, um ein zweites PCR-Produkt (150) zu erzeugen, oder optional
(a2") einen Einzelnukleotid-polymorphen zweites Allel-spezifischen Vorwärtsprimer (120), umfassend ein Einzelnukleotid-polymorphes zweites Allel-spezifisches Oligonukleotid (121), das spezifisch an die Zielnukleinsäure hybridisieren kann und an seinem 3'-Ende ein für das zweite Allel des Einzelnukleotid-Polymorphismus spezifisches Nukleotid aufweist, um ein zweites PCR-Produkt (150) zu erzeugen; und
(b) einen universellen Rückwärtsprimer (130) zur Amplifikation der Zielnukleinsäure; und
(c1) einen ersten Verlängerungsprimer (160), der mit dem Komplementären (113) des ersten 5'-Ende-Tag-Oligonukleotids (112) hybridisiert, umfassend:
c11) ein erstes 5'-Ende-Verlängerungspolynukleotid, das unter PCR-Bedingungen mit keiner der Zielnukleinsäuren, dem Einzelnukleotid-polymorphen erstes Allel-spezifischen Vorwärtsprimer (110) und dem Rückwärtsprimer (130) hybridisiert, wobei das erste Verlängerungspolynukleotid 20 bis 200 nt beträgt und zur Verlängerung des ersten PCR-Produkts (140) vorgesehen ist; und
c12) ein erstes 3'-Ende-Tag-Oligonukleotid, das mit dem Komplementären (113) des ersten 5'-Ende-Tag-Oligonukleotids (112) des ersten PCR-Produkts (140) hybridisiert; und
(d1) einen ersten Verlängerungs-Produktamplifikations-Vorwärtsprimer (200), der eine aus dem ersten Verlängerungspolynukleotid ausgewählte Nukleinsäuresequenz umfasst; und
(ii) Durchführung einer PCR der Reaktionslösung.

8. Verfahren nach Anspruch 7, wobei in (i), der Reaktionslösung ferner Folgendes zugesetzt wird:
(c2) ein zweiter Verlängerungsprimer (170), der mit dem Komplementären (123) des zweiten 5'-Ende-Tag-Oligonukleotids (122) hybridisiert, umfassend:
c21) ein zweites 5'-Ende-Verlängerungspolynukleotid, das unter PCR-Bedingungen mit keiner der Zielnukleinsäuren, dem Einzelukleotid-polymorphen erstes Allel-spezifischen Vorwärtsprimer (110), dem Einzelnukleotid-polymorphen zweites Allel-spezifischen Vorwärtsprimer (120) und dem Rückwärtsprimer (130) hybridisiert, wobei das zweite Verlängerungspolynukleotid eine Länge aufweist, die von der des ersten Verlängerungspolynukleotids in der Gelelektrophorese unterscheidbar ist, und wobei das zweite Verlängerungspolynucleotid zum Verlängern der Länge des zweiten PCR-Produkts (150) vorgesehen ist; und
c22) ein zweites 3'-Ende-Tag-Oligonukleotid, das mit dem Komplementären (123) des zweiten 5'-Ende-Tag-Oligonukleotids (122) des zweiten PCR-Produkts (150) hybridisiert; und
(d2) ein zweiter Verlängerungs-Produktamplifikations-Vorwärtsprimer (200), der eine aus dem zweiten Extensionspolynukleotid ausgewählte Nukleinsäuresequenz umfasst.

9. Verfahren zum Nachweis von Einzelnukleotid-Polymorphismus, umfassend:
(i) Durchführen einer Allel-spezifischen PCR einer Zielnukleinsäure unter Verwendung von:
(a1) einem Einzelnukleotid-polymorphen erstes Allel-spezifischen Vorwärtsprimer (110), umfassend:
ein erstes 5'-Ende-Tag-Oligonukleotid (112), das unter PCR-Bedingungen nicht an eine Zielnukleinsäure hybridisiert; und
ein Einzelnukleotid-polymorphes erstes Allel-spezifisches Oligonukleotid (111), das spezifisch mit der Zielnukleinsäure hybridisieren kann und an seinem 3 '-Ende ein Nukleotid aufweist, das spezifisch für das erste Allel des Einzelnukleotid-Polymorphismus ist, um ein erstes PCR-Produkt zu erzeugen (140);
(a2') einem Einzelnukleotid-polymorphen zweites Allel-spezifischen Vorwärtsprimer (120), umfassend:
ein zweites 5'-Ende-Tag-Oligonukleotid (122), das unter PCR-Bedingungen nicht mit der Zielnukleinsäure und dem ersten 5'-Ende-Tag-Oligonukleotid (112) hybridisiert; und
ein Einzelnukleotid-polymorphes zweites Allel-spezifisches Oligonukleotid (121), das spezifisch an die Zielnukleinsäure hybridisieren kann und an seinem 3'-Ende ein für das zweite Allel des Einzelnukleotid-Polymorphismus spezifisches Nukleotid aufweist, um ein zweites PCR-Produkt (150) zu erzeugen, oder optional
(a2") einem Einzelnukleotid-polymorphen zweites Allel-spezifischen Vorwärtsprimer (120), umfassend ein Einzelnukleotid-polymorphes zweites Allel-spezifisches Oligonukleotid (121), das spezifisch an die Zielnukleinsäure hybridisieren kann und an seinem 3'-Ende ein für das zweite Allel des Einzelnukleotid-Polymorphismus spezifisches Nukleotid aufweist, um ein zweites PCR-Produkt (150) zu erzeugen; und
(b) einem universellen Rückwärtsprimer (130) zur Amplifikation der Zielnukleinsäure; und (ii) Durchführung einer Primerverlängerung eines Produkts der Allel-spezifischen PCR unter Verwendung von
(c1) einem ersten Verlängerungsprimer (160), der mit dem Komplementären (113) des ersten 5'-Ende-Tag-Oligonukleotids (112) hybridisiert, umfassend:
c11) ein erstes 5'-Ende-Verlängerungspolynukleotid, das unter PCR-Bedingungen mit keiner der Zielnukleinsäuren, dem Einzelnukleotid-polymorphen erstes Allel-spezifischen Vorwärtsprimer (110) und dem Rückwärtsprimer (130) hybridisiert, wobei das erste Verlängerungspolynukleotid 20 bis 200 nt beträgt und zur Verlängerung des ersten PCR-Produkts (140) vorgesehen ist; und
c12) ein erstes 3'-Ende-Tag-Oligonukleotid, das mit dem Komplementären (113) des ersten 5'-Ende-Tag-Oligonukleotids (112) des ersten PCR-Produkts (140) hybridisiert.

10. Verfahren nach Anspruch 9, wobei in (ii), der Lösung ferner zugesetzt wird,
(c2) ein zweiter Verlängerungsprimer (170), der mit dem Komplementären (123) des zweiten 5'-Ende-Tag-Oligonukleotids (122) hybridisiert, umfassend:
c21) ein zweites 5'-Ende-Verlängerungspolynukleotid, das unter PCR-Bedingungen mit keiner der Zielnukleinsäuren, dem Einzelukleotid-polymorphen erstes Allel-spezifischen Vorwärtsprimer (110), dem Einzelnukleotid-polymorphen zweites Allel-spezifischen Vorwärtsprimer (120) und dem Rückwärtsprimer (130) hybridisiert, wobei das zweite Verlängerungspolynukleotid eine Länge aufweist, die von der des ersten Verlängerungspolynukleotids in der Gelelektrophorese unterscheidbar ist, und wobei das zweite Verlängerungspolynucleotid zum Verlängern der Länge des zweiten PCR-Produkts (150) vorgesehen ist; und
c22) ein zweites 3'-Ende-Tag-Oligonukleotid, das mit dem Komplementären (123) des zweiten 5'-Ende-Tag-Oligonukleotids (122) des zweiten PCR-Produkts (150) hybridisiert.

11. Verfahren nach Anspruch 10, ferner umfassend:
(iii) Durchführen einer PCR zur Amplifikation eines Produkts der Primerverlängerung unter Verwendung des Folgenden:
(d1) ein erster Verlängerungs-Produktamplifikations-Vorwärtsprimer (200), der eine Nukleinsäuresequenz umfasst, die aus dem ersten Verlängerungspolynukleotid ausgewählt ist; und
(d2) ein zweiter Verlängerungs-Produktamplifikations-Vorwärtsprimer (200), umfassend eine Nukleinsäuresequenz, die aus dem zweiten Verlängerungspolynukleotid ausgewählt ist; oder
(d3) ein Verlängerungs-Produktamplifikations-Vorwärtsprimer (200), umfassend eine Konsensussequenz aus dem ersten Verlängerungspolynukleotid und dem zweiten Verlängerungspolynukleotid; und
(b) den Rückwärtsprimer (130).

12. Verfahren nach Anspruch 7 oder 8, wobei (i) und (ii) in einem einzigen Schritt in einem einzigen Reaktor durchgeführt werden.

13. Verfahren nach Anspruch 11, wobei (i), (ii) und (iii) in einem einzigen Schritt in einem einzigen Reaktor durchgeführt werden.

## Revendications

1. Kit de détection de polymorphisme de simple nucléotide, comprenant :
(a1) une première amorce sens spécifique d'allèle polymorphique de simple nucléotide (110) comprenant :
un premier oligonucléotide étiquette à l'extrémité 5' (112) qui ne s'hybride pas à un nucléotide cible dans des conditions de PCR ; et
un premier oligonucléotide spécifique d'allèle polymorphique de simple nucléotide (111) qui est capable de s'hybrider spécifiquement à l'acide nucléique cible et a à son extrémité 3', un nucléotide spécifique pour le premier allèle du polymorphisme de simple nucléotide, pour produire un premier produit de PCR (140) ;
(a2') une seconde amorce sens spécifique d'allèle polymorphique de simple nucléotide (120) comprenant :
un second oligonucléotide étiquette à l'extrémité 5' (122) qui ne s'hybride pas à l'acide nucléotide cible et au premier oligonucléotide étiquette à l'extrémité 5' (112) dans des conditions de PCR ; et
un second oligonucléotide spécifique d'allèle polymorphique de simple nucléotide (121) qui est capable de s'hybrider spécifiquement à l'acide nucléique cible et a à son extrémité 3', un nucléotide spécifique pour le second allèle du polymorphisme de simple nucléotide, pour produire un second produit de PCR (1150), ou éventuellement
(a2")une seconde amorce sens spécifique d'allèle polymorphique de simple nucléotide (120) comprenant un second oligonucléotide spécifique d'allèle polymorphique de simple nucléotide (121) qui est capable de s'hybrider spécifiquement à l'acide nucléique cible et a à son extrémité 3', un nucléotide spécifique pour le second allèle du polymorphisme de simple nucléotide, pour produire un second produit de PCR (150) ; et
(b) une amorce antisens universelle (130) pour amplifier l'acide nucléique cible ; et
(c1) une première amorce d'extension (160) s'hybridant à la complémentarité (113) du premier oligonucléotide étiquette de l'extrémité 5' (112) comprenant :
c11) un premier polynucléotide d'extension qui ne s'hybride à aucun des acides nucléiques cibles, à la première amorce sens spécifique d'allèle polymorphique de simple nucléotide (110) et à l'amorce antisens (130) dans des conditions de PCR, le premier polynucléotide d'extension étant de 20 à 200 nt et fourni pour étendre la longueur du premier produit de PCR (140) ; et
c12) un premier oligonucléotide étiquette à l'extrémité 3' qui s'hybride à la complémentarité (113) du premier oligonucléotide étiquette à l'extrémité 5' (112) du premier produit de PCR (140).

2. Kit selon la revendication 1, comprenant en outre :
(c2) une seconde amorce d'extension (170) s'hybridant à la complémentarité (123) du second oligonucléotide étiquette à l'extrémité 5' (122) comprenant :
c21) un second polynucléotide d'extension à l'extrémité 5' qui ne s'hybride à aucun des acides nucléique cibles, à la première amorce sens spécifique d'allèle polymorphique de simple nucléotide (110), à la seconde amorce sens spécifique d'allèle polymorphique de simple nucléotide (120) et à l'amorce antisens (130) dans des conditions de PCR, le second polynucléotide d'extension ayant une longueur qui peut être distinguée de celle du premier polynucléotide d'extension par électrophorèse sur gel, et le second polynucléotide d'extension étant fourni pour étendre la longueur du second produit de PCR (150) ; et
c22) un second oligonucléotide étiquette à l'extrémité 3' qui s'hybride à la complémentarité (123) du second oligonucléotide étiquette à l'extrémité 5' (122) du second produit de PCR (150).

3. Kit selon la revendication 2, comprenant en outre :
(d1) une première amorce sens d'amplification de produit d'extension (200) comprenant une séquence d'acide nucléique choisie parmi le premier polynucléotide d'extension ; et
(d2) une seconde amorce sens d'amplification de produit d'extension (200) comprenant une séquence d'acide nucléique choisie parmi le second polynucléotide d'extension.

4. Kit selon la revendication 2, comprenant en outre :
(d3) une amorce sens d'amplification de produit d'extension (200) comprenant une séquence consensus du premier polynucléotide d'extension et du second polynucléotide d'extension.

5. Kit selon l'une quelconque des revendications 1 à 4, dans lequel le premier allèle est mutant et le second allèle est sauvage ; ou le premier allèle est sauvage et le second allèle est mutant.

6. Kit selon l'une quelconque des revendications 1 à 5, comprenant en outre un tampon de réaction, un mélange de dNTP et une ADN polymérase thermiquement stable.

7. Procédé de détection d'un polymorphisme de simple nucléotide, comprenant :
(i) l'addition, à une solution de réaction comprenant un échantillon d'acide nucléique cible à amplifier, d'un mélange de dNTP, d'une ADN polymérase thermiquement stable et d'un tampon de réaction,
(a1) une première amorce sens spécifique d'allèle polymorphique de simple nucléotide (110) comprenant : un premier oligonucléotide étiquette à l'extrémité 5' (112) qui ne s'hybride pas à un acide nucléique cible dans des conditions de PCR ; et
un premier oligonucléotide spécifique d'allèle polymorphique de simple nucléotide (111) qui est capable de s'hybrider spécifiquement à l'acide nucléique cible et a à son extrémité 3', un nucléotide spécifique pour le premier allèle du polymorphisme de simple nucléotide, pour produire un premier produit de PCR (140) ;
(a2')une seconde amorce sens spécifique d'allèle polymorphique de simple nucléotide (120) comprenant : un second oligonucléotide étiquette à l'extrémité 5' (122) qui ne s'hybride pas à l'acide nucléique cible et au premier oligonucléotide étiquette à l'extrémité 5' (112) dans des conditions de PCR ; et un second oligonucléotide spécifique d'allèle polymorphique de simple nucléotide (121) qui est capable de s'hybrider spécifiquement à l'acide nucléique cible et a à son extrémité 3', un nucléotide spécifique pour le second allèle du polymorphisme de simple nucléotide, pour produire un second produit de PCR (150), ou éventuellement
(a2")une seconde amorce sens spécifique d'allèle polymorphique de simple nucléotide (120) comprenant un second oligonucléotide spécifique d'allèle polymorphique de simple nucléotide (121) qui est capable de s'hybrider spécifiquement à l'acide nucléique cible et a à son extrémité 3', un nucléotide spécifique pour le second allèle du polymorphisme de simple nucléotide, pour produire un second produit de PCR (150) ; et
(b) une amorce antisens universelle (130) pour amplifier l'acide nucléique cible ;
(c1) une première amorce d'extension (160) s'hybridant à la complémentarité (113) du premier oligonucléotide étiquette à l'extrémité 5' (112) comprenant :
c11) un premier polynucléotide d'extension à l'extrémité 5' qui ne s'hybride à aucun des acides nucléiques cibles, à la première amorce sens spécifique d'allèle polymorphique de simple nucléotide (110) et à l'amorce inverse (130) dans des conditions de PCR, le premier polynucléotide d'extension étant de 20 à 200 nt et fourni pour étendre la longueur du premier produit de PCR (140) ; et
c12) un premier oligonucléotide étiquette à l'extrémité 3' qui s'hybride à la complémentarité (113) du premier oligonucléotide étiquette à extrémité 5' (112) du premier produit de PCR (140) ; et
(d1) une première amorce sens d'amplification de produit d'extension (200) comprenant une séquence d'acide nucléique choisie parmi le premier polynucléotide d'extension ; et
(ii) la réalisation de la PCR de la solution réactionnelle.

8. Procédé selon la revendication 7, dans lequel dans (i), l'addition en outre à la solution réactionnelle de ce qui suit :
(c2) une seconde amorce d'extension (170) s'hybridant à la complémentarité (123) du second oligonucléotide étiquette à l'extrémité 5' (122) comprenant :
c21) un second polynucléotide d'extension à l'extrémité 5' qui ne s'hybride à aucun des acides nucléiques cibles, à la première amorce sens spécifique d'allèle polymorphique de simple nucléotide (110), à la seconde amorce sens spécifique d'allèle polymorphique de simple nucléotide (120) et à l'amorce antisens (130) dans des conditions de PCR, le second polynucléotide d'extension ayant une longueur qui peut être distinguée de celle du premier polynucléotide d'extension par électrophorèse sur gel, et le second polynucléotide d'extension étant fourni pour étendre la longueur du second produit de PCR (150) ; et
c22) un second oligonucléotide étiquette à l'extrémité 3' qui s'hybride à la complémentarité (123) du second oligonucléotide étiquette à l'extrémité 5' (122) du second produit de PCR (150) ; et
(d2) une seconde amorce sens d'amplification de produit d'extension (200) comprenant une séquence d'acide nucléique choisie parmi le second polynucléotide d'extension.

9. Procédé de détection de polymorphisme de simple nucléotide, comprenant :
(i) la réalisation d'une PCR spécifique d'allèle d'un acide nucléique cible en utilisant :
(a1) une première amorce sens spécifique d'allèle polymorphique de simple nucléotide (110) comprenant : un premier oligonucléotide étiquette à l'extrémité 5' (112) qui ne s'hybride pas à un acide nucléique cible dans des conditions de PCR ; et
un premier oligonucléotide spécifique d'allèle polymorphique de simple nucléotide (111) qui est capable de s'hybrider spécifiquement à l'acide nucléique cible et a à son extrémité 3', un nucléotide spécifique pour le premier allèle du polymorphisme de simple nucléotide, pour produire un premier produit de PCR (140) ;
(a2') une seconde amorce sens spécifique d'allèle polymorphique de simple nucléotide (120) comprenant :
un second oligonucléotide étiquette à l'extrémité 5' (122) qui ne s'hybride pas à l'acide nucléique cible et au premier oligonucléotide étiquette à l'extrémité 5' (112) dans des conditions de PCR ; et
un second olignonucléotide spécifique d'allèle polymorphique de simple nucléotide (121) qui est capable de s'hybrider spécifiquement à l'acide nucléique cible et a à son extrémité 3', un nucléotide spécifique pour le second allèle du polymorphisme de simple nucléotide, pour produire un second produit de PCR (150), ou éventuellement
(a2") une seconde amorce sens spécifique d'allèle polymorphique de simple nucléotide (120) comprenant un second oligonucléotide spécifique d'allèle polymorphique de simple nucléotide (121) qui est capable de s'hybrider spécifiquement à l'acide nucléique cible et a à son extrémité 3', un nucléotide spécifique pour le second allèle du polymorphisme de simple nucléotide, pour produire un second produit de PCR (150) ; et
(b) une amorce inverse universelle (130) pour amplifier l'acide nucléique cible ; et
(ii) la réalisation de l'extension d'amorce d'un produit de la PCR spécifique d'allèle par l'utilisation de
(c1) une première amorce d'extension (160) s'hybridant à la complémentarité (113) du premier oligonucléotide étiquette à l'extrémité 5' (112) comprenant :
c11) un premier polynucléotide d'extension à l'extrémité 5' qui ne s'hybride à aucun des acides nucléiques cibles, à la première amorce sens spécifique d'allèle polymorphique de simple nucléotide (110) et à l'amorce antisens (130) dans des conditions de PCR, le premier polynucléotide d'extension étant de 20 à 200 nt et fourni pour étendre la longueur du premier produit de PCR (140) ; et
c12) un premier oligonucléotide étiquette à l'extrémité 3' qui s'hybride à la complémentarité (113) du premier oligonucléotide étiquette à l'extrémité 5' (112) du premier produit de PCR (140).

10. Procédé selon la revendication 9, dans lequel dans (ii), l'addition en outre à la solution,
(c2) d'une seconde amorce d'extension (170) s'hybridant à la complémentarité (123) du second oligonucléotide étiquette à l'extrémité 5' (122) comprenant :
c21) un second polynucléotide d'extension à l'extrémité 5' qui ne s'hybride à aucun des acides nucléiques cibles, à la première amorce sens spécifique d'allèle polymorphique de simple nucléotide (110), à la seconde amorce sens spécifique d'allèle polymorphique de simple nucléotide (120), et à l'amorce antisens (130) dans des conditions de PCR, le second polynucléotide d'extension ayant une longueur qui peut être distinguée de celle du premier polynucléotide d'extension par électrophorèse sur gel, et le second polynucléotide d'extension étant fourni pour étendre la longueur du second produit de PCR (150) ; et
c22) un second oligonucléotide étiquette à l'extrémité 3' qui s'hybride à la complémentarité (123) du second oligonucléotide étiquette à l'extrémité 5' (122) du second produit de PCR (150).

11. Procédé selon la revendication 10, comprenant en outre :
(iii) la réalisation d'une PCR pour l'amplification d'un produit de l'extension d'amorce, par l'utilisation de ce qui suit :
(d1) une première amorce sens d'amplification de produit d'extension (200) qui comprend une séquence d'acide nucléique choisie parmi le premier polynucléotide d'extension, et
(d2) une seconde amorce sens d'amplification de produit d'extension (200) comprenant une séquence d'acide nucléique choisie parmi le second polynucléotide d'extension ; ou
(d3) une amorce sens d'amplification de produit d'extension (200) comprenant une séquence consensus du premier polynucléotide d'extension et du second polynucléotide d'extension ;
et
(b) l'amorce inverse (130).

12. Procédé selon la revendication 7 ou 8, dans lequel (i) et (ii) sont effectuées par une seule étape dans un seul réacteur.

13. Procédé selon la revendication 11, dans lequel (i), (ii) et (iii) sont effectuées par une seule étape dans un seul réacteur.


<110> HeimBiotek Inc.
<120> A kit and method for detecting single nucleotide polymorphic DNA
<130> PT16-5142
<160> 20
<170> KopatentIn 2.0
<210> 1
   <211> 3913
   <212> DNA
   <213> Homo sapiense
<400> 1
   agcgcccaat acgcaaaccg cctctccccg cgcgttggcc gattcattaa tgcagctggc 60
   acgacaggtt tcccgactgg aaagcgggca gtgagcgcaa cgcaattaat gtgagttagc 120
   tcactcatta ggcaccccag gctttacact ttatgcttcc ggctcgtatg ttgtgtggaa 180
   ttgtgagcgg ataacaattt cacacaggaa acagctatga ccatgattac gccaagcttg 240
   gtaccgagct cggatccact agtaacggcc gccagtgtgc tggaattcgc ccttggagcc 300
   cccgttctat atcatcaytg agttcatgac ctacgggaac ctcctggact acctgaggga 360
   gtgcaaccgg caggaggtga acgccgtggt gctgctgtac aagggcgaat tctgcagata 420
   tccatcacac tggcggccgc tcgagcatgc atctagaggg cccaattcgc cctatagtga 480
   atcgtattac aattcactgg ccgtcgtttt acaacgtcgt gactgggaaa accctggcgt 540
   tacccaactt aatcgccttg cagcacatcc ccctttcgcc agctggcgta atagcgaaga 600
   ggcccgcacc gatcgccctt cccaacagtt gcgcagccta tacgtacggc agtttaaggt 660
   ttacacctat aaaagagaga gccgttatcg tctgtttgtg gatgtacaga gtgatattat 720
   tgacacgccg gggcgacgga tggtgatccc cctggccagt gcacgtctgc tgtcagataa 780
   agtctcccgt gaactttacc cggtggtgca tatcggggat gaaagctggc gcatgatgac 840
   caccgatatg gccagtgtgc cggtctccgt tatcggggaa gaagtggctg atctcagcca 900
   ccgcgaaaat gacatcaaaa acgccattaa cctgatgttc tggggaatat aaatgtcagg 960
   catgagatta tcaaaaagga tcttcaccta gatccttttc acgtagaaag ccagtccgca 1020
   gaaacggtgc tgaccccgga tgaatgtcag ctactgggct atctggacaa gggaaaacgc 1080
   aagcgcaaag agaaagcagg tagcttgcag tgggcttaca tggcgatagc tagactgggc 1140
   ggttttatgg acagcaagcg aaccggaatt gccagctggg gcgccctctg gtaaggttgg 1200
   gaagccctgc aaagtaaact ggatggcttt cttgccgcca aggatctgat ggcgcagggg 1260
   atcaagatct gatcaagaga caggatgagg atcgtttcgc atgattgaac aagatggatt 1320
   gcacgcaggt tctccggccg cttgggtgga gaggctattc ggctatgact gggcacaaca 1380
   gacaatcggc tgctctgatg ccgccgtgtt ccggctgtca gcgcaggggc gcccggttct 1440
   ttttgtcaag accgacctgt ccggtgccct gaatgaactg caggacgagg cagcgcggct 1500
   atcgtggctg gccacgacgg gcgttccttg cgcagctgtg ctcgacgttg tcactgaagc 1560
   gggaagggac tggctgctat tgggcgaagt gccggggcag gatctcctgt catcccacct 1620
   tgctcctgcc gagaaagtat ccatcatggc tgatgcaatg cggcggctgc atacgcttga 1680
   tccggctacc tgcccattcg accaccaagc gaaacatcgc atcgagcgag cacgtactcg 1740
   gatggaagcc ggtcttgtcg atcaggatga tctggacgaa gagcatcagg ggctcgcgcc 1800
   agccgaactg ttcgccaggc tcaaggcgcg catgcccgac ggcgaggatc tcgtcgtgac 1860
   ccatggcgat gcctgcttgc cgaatatcat ggtggaaaat ggccgctttt ctggattcat 1920
   cgactgtggc cggctgggtg tggcggaccg ctatcaggac atagcgttgg ctacccgtga 1980
   tattgctgaa gagcttggcg gcgaatgggc tgaccgcttc ctcgtgcttt acggtatcgc 2040
   cgctcccgat tcgcagcgca tcgccttcta tcgccttctt gacgagttct tctgaattga 2100
   aaaaggaaga gtatgagtat tcaacatttc cgtgtcgccc ttattccctt ttttgcggca 2160
   ttttgccttc ctgtttttgc tcacccagaa acgctggtga aagtaaaaga tgctgaagat 2220
   cagttgggtg cacgagtggg ttacatcgaa ctggatctca acagcggtaa gatccttgag 2280
   agttttcgcc ccgaagaacg ttttccaatg atgagcactt ttaaagttct gctatgtggc 2340
   gcggtattat cccgtattga cgccgggcaa gagcaactcg gtcgccgcat acactattct 2400
   cagaatgact tggttgagta ctcaccagtc acagaaaagc atcttacgga tggcatgaca 2460
   gtaagagaat tatgcagtgc tgccataacc atgagtgata acactgcggc caacttactt 2520
   ctgacaacga tcggaggacc gaaggagcta accgcttttt tgcacaacat gggggatcat 2580
   gtaactcgcc ttgatcgttg ggaaccggag ctgaatgaag ccataccaaa cgacgagcgt 2640
   gacaccacga tgcctgtagc aatggcaaca acgttgcgca aactattaac tggcgaacta 2700
   cttactctag cttcccggca acaattaata gactggatgg aggcggataa agttgcagga 2760
   ccacttctgc gctcggccct tccggctggc tggtttattg ctgataaatc tggagccggt 2820
   gagcgtgggt ctcgcggtat cattgcagca ctggggccag atggtaagcc ctcccgtatc 2880
   gtagttatct acacgacggg gagccaggca actatggatg aacgaaatag acagatcgct 2940
   gagataggtg cctcactgat taagcattgg taactgtcag accaagttta ctcatatata 3000
   ctttagattg atttaaaact tcatttttaa tttaaaagga tctaggtgaa gatccttttt 3060
   gataatctca tgaccaaaat cccttaacgt gagttttcgt tccactgagc gtcagacccc 3120
   gtagaaaaga tcaaaggatc ttcttgagat cctttttttc tgcgcgtaat ctgctgcttg 3180
   caaacaaaaa aaccaccgct accagcggtg gtttgtttgc cggatcaaga gctaccaact 3240
   ctttttccga aggtaactgg cttcagcaga gcgcagatac caaatactgt tcttctagtg 3300
   tagccgtagt taggccacca cttcaagaac tctgtagcac cgcctacata cctcgctctg 3360
   ctaatcctgt taccagtggc tgctgccagt ggcgataagt cgtgtcttac cgggttggat 3420
   tcaagacgat agttaccgga taaggcgcag cggtcgggct gaacgggggg ttcgtgcaca 3480
   cagcccagct tggagcgaac gacctacacc gaactgagat acctacagcg tgagctatga 3540
   gaaagcgcca cgcttcccga agggagaaag gcggacaggt atccggtaag cggcagggtc 3600
   ggaacaggag agcgcacgag ggagcttcca gggggaaacg cctggtatct ttatagtcct 3660
   gtcgggtttc gccacctctg acttgagcgt cgatttttgt gatgctcgtc aggggggcgg 3720
   agcctatgga aaaacgccag caacgcggcc tttttacggt tcctggcctt ttgctggcct 3780
   tttgctcaca tgttctttcc tgcgttatcc cctgattctg tggataaccg tattaccgcc 3840
   tttgagtgag ctgataccgc tcgccgcagc cgaacgaccg agcgcagcga atcagtgagc 3900
   gaggaagcgg aag 3913
<210> 2
   <211> 21
   <212> DNA
   <213> Artificial Sequence
<220>
   <223> C-specific forward primer
<400> 2
   gcccccgttc tatatcataa c 21
<210> 3
   <211> 21
   <212> DNA
   <213> Artificial Sequence
<220>
   <223> T-specific forward primer
<400> 3
   gcccccgttc tatatcataa t 21
<210> 4
   <211> 20
   <212> DNA
   <213> Artificial Sequence
<220>
   <223> reverse primer
<400> 4
   gaattcgccc ttgtacagca 20
<210> 5
   <211> 1200
   <212> DNA
   <213> Artificial Sequence
<220>
   <223> pBR322 plasmid
<400> 5
<210> 6
   <211> 45
   <212> DNA
   <213> Artificial Sequence
<220>
   <223> Tag complementary-forward primer
<400> 6
   cacggagtag ccgttatctg cagtttcatt tgatgctcga tgagt 45
<210> 7
   <211> 22
   <212> DNA
   <213> Artificial Sequence
<220>
   <223> reverse primer for extension polynucleotide
<400> 7
   agccgccgtc ccgtcaagtc ag 22
<210> 8
   <211> 18
   <212> DNA
   <213> Artificial Sequence
<220>
   <223> homologous sequence to pBR322
<400> 8
   cacggagtag ccgttatc 18
<210> 9
   <211> 39
   <212> DNA
   <213> Artificial Sequence
<220>
   <223> Mutant allele-specific extension primer
<400> 9
   gataacggct actccgtggc ccccgttcta tatcataat 39
<210> 10
   <211> 18
   <212> DNA
   <213> Artificial Sequence
<220>
   <223> a second tag oligonucleotide
<400> 10
   gataacggct actccgtg 18
<210> 11
   <211> 50
   <212> DNA
   <213> Artificial Sequence
<220>
   <223> wildtype specific forward primer for UGT1A1*28
<400> 11
   gacgagtcgg aatcgcaggt tcgccctctc ctacttatat atatattgtg 50
<210> 12
   <211> 54
   <212> DNA
   <213> Artificial Sequence
<220>
   <223> mutant specific forward primer for UGT1A1*28
<400> 12
   ccatgagtga cgactgaatc cgttcgccct ctcctactta tatatatata ttta 54
<210> 13
   <211> 20
   <212> DNA
   <213> Artificial Sequence
<220>
   <223> reverse primer for UGT1A1*28
<400> 13
   ctctgaaagt gaactccctg 20
<210> 14
   <211> 50
   <212> DNA
   <213> Artificial Sequence
<220>
   <223> wildtype specific exension primer for UGT1A1*28
<400> 14
   cgcacctcca ggaccaatct tgttcgggaa aggacgagtc ggaatcgcag 50
<210> 15
   <211> 100
   <212> DNA
   <213> Artificial Sequence
<220>
   <223> wildtype specific extension primer for UGT1A1*28
<400> 15
   cgcacctcca ggaccaatct tgttcgggaa aggacgagtc ggaatcgcag agttccgtcg 60
   acgatggctc catcgagtcc catgagtgac gactgaatcc 100
<210> 16
   <211> 25
   <212> DNA
   <213> Artificial Sequence
<220>
   <223> forward primer for exension product of UGT1A1*28
<400> 16
   cgcacctcca ggaccaatct tgttc 25
<210> 17
   <211> 42
   <212> DNA
   <213> Artificial Sequence
<220>
   <223> wildtype specific forward primer for UGT1A1*6
<400> 17
   ccatgagtga cgactgaatc cgaaaaggca atgagcatgg tt 42
<210> 18
   <211> 20
   <212> DNA
   <213> Artificial Sequence
<220>
   <223> reverse primer for UGT1A1*6
<400> 18
   ccacttactg cacaacaagg 20
<210> 19
   <211> 39
   <212> DNA
   <213> Artificial Sequence
<220>
   <223> mutant specific forward primer for UGT1A1^{∗}6
<400> 19
   gacgagtcgg aatcgcagga aaaggcaatg agcatggtc 39
<210> 20
   <211> 66
   <212> DNA
   <213> Artificial Sequence
<220>
   <223> mutant specific extension primer for UGT1A1^{∗}6
<400> 20
   cgcacctcca ggaccaatct tgttcgggaa aggacgagtc ggaatcgcag agttccgtcg 60
   acgatg 66